# EUROPÄISCHE PATENTANMELDUNG

(11) **EP 3 725 276 A1**
(43) Veröffentlichungstag der Anmeldung: **21.10.2020**
(21) Anmeldenummer: 19169907.3
(22) Anmeldetag: 17.04.2019
(51) Int. Cl.: A61F 9/008, A61F 9/009

(54) **OPHTHALMOLOGISCHE VORRICHTUNG ZUM BEARBEITEN EINES AUGES**

(71) Anmelder: Ziemer Ophthalmic Systems AG, 2562 Port (CH)
(72) Erfinder: RATHJEN, Christian, 28197 Bremen (DE)
(74) Vertreter: Rentsch Partner AG

(57) **Zusammenfassung**

Eine ophthalmologische Vorrichtung (1) zum Bearbeiten eines Auges (2) umfasst eine Laserquelle (11), ein Scannersystem (17) und einen Applikationskopf (14) mit einer Fokussieroptik (12) und einem Patienteninterface (13) zum Andocken des Applikationskopfes (14) am Auge (2). Die ophthalmologische Vorrichtung (1) umfasst überdies ein Messsystem (16) zum optischen Erfassen von Augenstrukturen, im am Auge (2) angedockten Zustand Applikationskopfes (14), und einen Schaltkreis (10), der eingerichtet ist, aus den erfassten Augenstrukturen ringförmig um die Zentrumsachse (v) der Vorderkammer (VK) des Auges (2) angeordnete Referenzstrukturen des Auges (2) zu ermitteln und ein definiertes dreidimensionales Bearbeitungsmodell bezüglich dieser Referenzstrukturen (VK) anzuordnen, um gemäss dem angeordneten dreidimensionalen Bearbeitungsmodell im Auge (2) ein dreidimensionales Bearbeitungsmuster (3*) zu bearbeiten.

## Beschreibung

### Technisches Gebiet

Die vorliegende Erfindung betrifft eine ophthalmologische Vorrichtung zum Bearbeiten eines Auges. Die vorliegende Erfindung betrifft insbesondere eine ophthalmologische Vorrichtung zum Bearbeiten eines Auges mittels eines gepulsten Laserstrahls gemäss einem dreidimensionalen Bearbeitungsmodell, wobei die ophthalmologische Vorrichtung einen Applikationskopf mit einer Fokussieroptik und einem Patienteninterface zum Fixieren des Applikationskopfs am Auge umfasst.

### Stand der Technik

Für die refraktive Korrektur eines Auges, bei welcher die Brechkraft des Auges verändert wird, werden ophthalmologische Lasersysteme eingesetzt, welche das Augengewebe, typischerweise die Hornhaut (Cornea) und/oder die Augenlinse, mittels eines fokussierten gepulsten Laserstrahls gemäss einem dreidimensionalen Schnittmodell bearbeiten. Um das dreidimensionale Schnittmodell in einer vorbereitenden Planungs- oder Untersuchungsphase zu erstellen und später bei der Laserbehandlung des Auges zum Erzeugen des entsprechenden dreidimensionalen Schnittmusters im zu korrigierenden Auge anzuwenden, hat der Patient mit dem betreffenden Auge ein Fixiertarget anzusehen, um eine definierte Ausrichtung des Auges sowohl zur Mess- oder Planungsvorrichtung als auch später zum bearbeitenden ophthalmologischen Lasersystem zu erstellen.

Ein Verfahren für die Vermessung der Cornea-Topografie und die Ausrichtung bei chirurgischen Behandlungen der Cornea, bei welchem das Auge eines Patienten an ein Patienteninterface angedockt wird, wenn der Patient den Blick auf ein Fixierlicht richtet und das Auge zur Achse des behandelnden Lasersystems ausgerichtet ist, wird in US 2015/0018674 beschrieben.

Bei der Vermessung oder Diagnose des Auges werden sowohl Messdaten des Auges als auch Referenzmerkmale bestimmt, beispielsweise auf dem Auge erzeugte Referenzmarkierungen, wie z.B. Lichtreflexe (insbesondere Purkinjereflexe), die in einer Aufsicht des Auges (Frontalansicht) aufgrund von Lichteinstrahlung durch die Mess- oder Planungsvorrichtung erzeugt werden, oder physiologische Merkmale des Auges, wie z.B. Adern in der Sklera, der Limbus, die Iris und die Pupille oder Merkmale der Retina, respektive auf das Auge aufgebrachte Farbmarkierungen. Das dreidimensionale Schnittmodell wird basierend auf den erfassten Messdaten und bezüglich der erfassten Referenzmerkmale definiert.

EP 2 236 109 beschreibt eine Planungsvorrichtung mit einem Referenzgenerator zum Definieren und Speichern einer geometrischen Referenz mit Bezug zu einem dreidimensionalen Augenmodell, einem Schnittflächeneditor zum Definieren und Positionieren von Schnittflächen im dreidimensionalen Augenmodell und einem Schnittmustergenerator zum Generieren von dreidimensionalen Schnittmustern für im Auge mittels Femtosekundenlaserpulsen auszuführenden Gewebeschnitten.

Bei der Behandlung durch das bearbeitende ophthalmologische Lasersystem wird dieselbe Situation wie bei der Vermessung respektive Diagnose reproduziert. D.h. der Patient richtet den Blick des Auges wieder auf ein entsprechend angeordnetes Fixiertarget und dann werden (vorzugweise die gleichen) Merkmale in situ bestimmt, also unter dem bearbeitenden ophthalmologischen Lasersystem. Werden die mit den Messdaten bestimmten und für das dreidimensionale Schnittmodell verwendeten Referenzmerkmale des Auges mit den entsprechenden Merkmalen in situ, bei der Behandlung, in Übereinstimmung gebracht, kann im Augengewebe ein dreidimensionales Schnittmuster erzeugt werden, das in Form und Grösse dem dreidimensionalen Schnittmodell entspricht und im Augengewebe bezüglich der Referenzmerkmale positioniert ist.

Bei den Verfahren, bei welchen der Patient sein Auge in der Vermessungs-/Planungsphase und in der Behandlungsphase auf ein Fixiertarget ausrichtet, besteht allerdings das Problem, dass wenn der Patient in der Vermessungs-/Planungsphase und/oder in der Behandlungsphase die Blickrichtung des Auges unterschiedlich oder schlecht auf das Fixiertarget ausrichtet, Positionierungs- und Ausrichtungsfehler des zu schneidenden Schnittmusters entstehen können. Diese Fehler können überdies auch dadurch verursacht werden, dass z.B. das Auge unter dem Lasersystem nicht gut positioniert ist und/oder vor oder während dem Andockvorgang an ein Patienteninterface verrutscht, wonach es nicht mehr fixiert. Das Auge ist dann bezüglich des Patienteninterfaces und damit auch bezüglich des Lasersystems verdreht respektive verkippt, da das Patienteninterface koaxial mit der optischen Achse des Lasersystems angeordnet ist. Weiterhin können beim Kontakt des Auges mit dem Patienteninterface Referenzmarkierungen in Abhängigkeit vom Lasersystem nicht mehr sichtbar sein, da sich die optische Abbildung verändert.

### Darstellung der Erfindung

Es ist eine Aufgabe der vorliegenden Erfindung eine ophthalmologische Vorrichtung zum Bearbeiten eines Auges vorzuschlagen, welche zumindest einige Nachteile des Stands der Technik nicht aufweist. Es ist insbesondere eine Aufgabe der vorliegenden Erfindung eine ophthalmologische Vorrichtung zum Bearbeiten eines Auges vorzuschlagen, welche bei der Bearbeitung nicht zwingend eine Fixierung der Blickausrichtung auf ein Fixiertarget erfordert.

Gemäss der vorliegenden Erfindung werden diese Ziele durch die Merkmale des unabhängigen Anspruchs erreicht. Weitere vorteilhafte Ausführungsformen gehen ausserdem aus den abhängigen Ansprüchen und der Beschreibung hervor.

Eine ophthalmologische Vorrichtung zum Bearbeiten eines Auges umfasst eine Laserquelle, die eingerichtet ist, einen gepulsten Laserstrahl zu erzeugen; einen Applikationskopf mit einer Fokussieroptik und einem Patienteninterface, wobei die Fokussieroptik eingerichtet ist, den gepulsten Laserstrahl im Auge zu fokussieren, und wobei das Patienteninterface eingerichtet ist, den Applikationskopf am Auge zu fixieren; einen Schaltkreis, der eingerichtet ist, ein dreidimensionales Bearbeitungsmodell eines im Auge zu bearbeitenden dreidimensionalen Bearbeitungsmusters zu speichern; ein Scannersystem, das eingerichtet ist, den gepulsten Laserstrahl gemäss dem dreidimensionalen Bearbeitungsmodell auf Bearbeitungszielpunkte des im Auge zu bearbeitenden dreidimensionalen Bearbeitungsmusters zu lenken; und ein Messsystem, das eingerichtet ist, in einem am Auge fixierten Zustand des Applikationskopfs Strukturen des Auges optisch zu erfassen. In einer Ausführungsvariante ist das Messsystem eingerichtet, dreidimensionale Augenstrukturen zu erfassen.

Die oben genannten Ziele werden durch die vorliegende Erfindung in einem ersten Aspekt der Erfindung insbesondere dadurch erreicht, dass der Schaltkreis eingerichtet ist, aufgrund der durch das Messsystem im am Auge fixierten Zustand des Applikationskopfs optisch erfassten Strukturen Referenzstrukturen des Auges zu ermitteln, welche im Wesentlichen ringförmig um eine Zentrumsachse der Vorderkammer des Auges angeordnet sind, und dass der Schaltkreis eingerichtet ist, einen Positionierungsreferenzpunkt, zur Positionierung des dreidimensionalen Bearbeitungsmodells im Auge, zu speichern, unter Verwendung der Referenzstrukturen einen im am Auge fixierten Zustand des Applikationskopfs verschobenen Positionierungsreferenzpunkt zu bestimmen, das dreidimensionale Bearbeitungsmodell im am Auge fixierten Zustand des Applikationskopfs bezüglich des verschobenen Positionierungsreferenzpunkts zu positionieren, und das Scannersystem im am Auge fixierten Zustand des Applikationskopfs derart zu steuern, dass das Scannersystem den gepulsten Laserstrahl gemäss dem bezüglich des verschobenen Positionierungsreferenzpunkts positionierten dreidimensionalen Bearbeitungsmodell auf die Bearbeitungszielpunkte des im Auge zu bearbeitenden dreidimensionalen Bearbeitungsmusters lenkt.

Die oben genannten Ziele werden durch die vorliegende Erfindung in einem zweiten Aspekt der Erfindung insbesondere dadurch erreicht, dass der Schaltkreis eingerichtet ist, aufgrund der durch das Messsystem im am Auge fixierten Zustand des Applikationskopfs optisch erfassten Strukturen Referenzstrukturen des Auges zu ermitteln, welche im Wesentlichen ringförmig um eine Zentrumsachse der Vorderkammer des Auges angeordnet sind, und dass der Schaltkreis eingerichtet ist, das dreidimensionale Bearbeitungsmodell bezüglich der optisch erfassten Referenzstrukturen auszurichten, und das Scannersystem im am Auge fixierten Zustand des Applikationskopfs derart zu steuern, dass das Scannersystem den gepulsten Laserstrahl gemäss dem bezüglich der optisch erfassten Referenzstrukturen ausgerichteten dreidimensionalen Bearbeitungsmodell auf die Bearbeitungszielpunkte des im Auge zu bearbeitenden dreidimensionalen Bearbeitungsmusters lenkt.

An dieser Stelle soll angeführt werden, dass mit dem Begriff "Ausrichtung" oder "ausrichten" eine richtungsmässige Anordnung und Orientierung eines zwei- oder dreidimensionalen Objekts bezüglich eines räumlichen Bezugsystems zu verstehen ist. Wenn beispielsweise für die erfassten Referenzstrukturen eine Referenzebene und eine Referenzachse, z.B. eine Zentrumsachse, definiert werden, dann kann die Ausrichtung respektive die richtungsmässige Anordnung und Orientierung des dreidimensionalen Bearbeitungsmodells mittels Verkippungs- und Verdrehungswinkeln bezüglich dieser Referenzebene und/oder dieser Referenzachse definiert werden.

In einer Ausführungsvariante des zweiten Aspekts der Erfindung ist der Schaltkreis eingerichtet, einen Positionierungsreferenzpunkt, zur Positionierung des dreidimensionalen Bearbeitungsmodells im Auge, zu speichern, unter Verwendung der Referenzstrukturen einen im am Auge fixierten Zustand des Applikationskopfs verschobenen Positionierungsreferenzpunkt zu bestimmen, das dreidimensionale Bearbeitungsmodell im am Auge fixierten Zustand des Applikationskopfs bezüglich des verschobenen Positionierungsreferenzpunkts zu positionieren, und das Scannersystem im am Auge fixierten Zustand des Applikationskopfs derart zu steuern, dass das Scannersystem den gepulsten Laserstrahl gemäss dem bezüglich des verschobenen Positionierungsreferenzpunkts positionierten dreidimensionalen Bearbeitungsmodell auf die Bearbeitungszielpunkte des im Auge zu bearbeitenden dreidimensionalen Bearbeitungsmusters lenkt.

An dieser Stelle soll angeführt werden, dass mit dem Begriff "Positionierung" oder "positionieren" eine Anordnung eines Objekts bezüglich eines Punkts in einem räumlichen Bezugsystem zu verstehen ist. Wenn beispielsweise zur Positionierung des dreidimensionalen Bearbeitungsmodells im Auge ein Positionierungsreferenzpunkt für einen Zentrumspunkt (oder einen anderen Bezugspunkt) des dreidimensionalen Bearbeitungsmodells definiert wird, dann ist das Bearbeitungsmodell im Auge bezüglich dieses Positionierungsreferenzpunkts positioniert, wenn sein Zentrumspunkt (respektive sein anderer Bezugspunkt) auf dem Positionierungsreferenzpunkt angeordnet ist (eine bestimmte Ausrichtung des dreidimensionalen Bearbeitungsmodells ist dadurch jedoch nicht definiert).

Die nachfolgend beschriebenen Ausführungsvarianten sind auf den ersten und den zweiten Aspekt der Erfindung anwendbar.

In einer Ausführungsvariante ist der Schaltkreis eingerichtet, aufgrund der optisch erfassten Augenstrukturen dreidimensionale Referenzstrukturen des Auges zu ermitteln.

In einer Ausführungsvariante ist der Schaltkreis eingerichtet, den Positionierungsreferenzpunkt mit Referenzmessdaten zur Positionierung des Positionierungsreferenzpunkts bezüglich der Referenzstrukturen in einem nicht am Auge fixierten Zustand des Applikationskopfs zu speichern, und den verschobenen Positionierungsreferenzpunkt im am Auge fixierten Zustand des Applikationskopfs unter Verwendung der Referenzmessdaten und der im am Auge fixierten Zustand des Applikationskopfs ermittelten Referenzstrukturen zu bestimmen.

In einer Ausführungsvariante umfassen die Referenzmessdaten Distanzangaben vom Positionierungsreferenzpunkt zu mindestens zwei gegenüberliegenden Punkten auf den im Wesentlichen ringförmig angeordneten Referenzstrukturen im nicht am Auge fixierten Zustand des Applikationskopfs, und der Schaltkreis ist eingerichtet, den verschobenen Positionierungsreferenzpunkt im am Auge fixierten Zustand des Applikationskopfs unter Verwendung der Distanzangaben bezüglich der mindestens zwei gegenüberliegenden Punkten auf den im am Auge fixierten Zustand des Applikationskopfs ermittelten Referenzstrukturen zu bestimmen.

In einer Ausführungsvariante definieren die Distanzangaben Weglängen vom Positionierungsreferenzpunkt entlang erfasster Augenstrukturen des Auges zu den mindestens zwei gegenüberliegenden Punkten auf den im Wesentlichen ringförmig angeordneten Referenzstrukturen.

In einer Ausführungsvariante umfassen die Distanzangaben Weglängen entlang einer neutralen Faser der Cornea, der Corneavorderfläche und/oder der Cornearückfläche.

In einer Ausführungsvariante ist der Schaltkreis eingerichtet, den verschobenen Positionierungsreferenzpunkt im am Auge fixierten Zustand des Applikationskopfs unter Verwendung eines Verhältnisses der in den Referenzmessdaten enthaltenen Distanzangaben bestimmt.

In einer Ausführungsvariante umfassen die Referenzmessdaten eine Distanzangabe und einen Projektionspunkt des Positionierungsreferenzpunkts bezüglich einer Ebene, welche durch im nicht am Auge fixierten Zustand des Applikationskopfs erfasste Referenzstrukturen definiert ist; und der Schaltkreis ist eingerichtet, den verschobenen Positionierungsreferenzpunkt im am Auge fixierten Zustand des Applikationskopfs unter Verwendung der Distanzangabe und des Projektionspunkts des Positionierungsreferenzpunkts bezüglich der Referenzebene im am Auge fixierten Zustand des Applikationskopfs zu bestimmen.

In einer Ausführungsvariante ist der Schaltkreis eingerichtet, aufgrund der ermittelten Referenzstrukturen eine durch die Referenzstrukturen definierte Zentrumsachse der Vorderkammer zu bestimmen, und das dreidimensionale Bearbeitungsmodell bezüglich der Zentrumsachse der Vorderkammer auszurichten.

In einer Ausführungsvariante ist der Schaltkreis eingerichtet, eine durch die Referenzstrukturen verlaufende Referenzebene zu bestimmen, und das dreidimensionale Bearbeitungsmodell bezüglich der Referenzebene auszurichten.

In einer Ausführungsvariante umfassen die Referenzstrukturen den Limbus, die Iris, den Vorderkammerwinkel, den Skleral Spur, den Schlemmschen Kanal und/oder die Schwalbes Linie.

In einer Ausführungsvariante ist der Schaltkreis eingerichtet, das dreidimensionale Bearbeitungsmodell über eine Kommunikationsstrecke von einer externen Planungsvorrichtung zu empfangen.

In einer Ausführungsvariante ist das Messsystem eingerichtet, die Strukturen des Auges über einen in Richtung einer optischen Achse der Fokussieroptik verlaufenden Tiefenbereich optisch zu erfassen.

In einer Ausführungsvariante ist das Messsystem als interferometrisches Messsystem ausgeführt, und der Schaltkreis ist eingerichtet, das Messsystem derart zu steuern, dass das Messsystem im am Auge fixierten Zustand des Applikationskopfs die Strukturen des Auges optisch erfasst, und die Referenzstrukturen aus den optisch erfassten Strukturen zu ermitteln. Mittels des interferometrischen Messsystems können dreidimensionale Augenstrukturen und Referenzstrukturen ermittelt werden.

In einer Ausführungsvariante ist das Messsystem eingerichtet, die Strukturen des Auges in einem oder mehreren Querschnittsbildern des Auges zu erfassen, und der Schaltkreis ist eingerichtet, die Referenzstrukturen des Auges aus dem einen oder den mehreren Querschnittsbildern des Auges zu ermitteln. Über die Erfassung und Verwendung mehrerer Querschnittsbilder können dreidimensionale Augenstrukturen und Referenzstrukturen ermittelt werden.

In einer Ausführungsvariante umfasst das Patienteninterface einen Kontaktkörper, welcher im am Auge fixierten Zustand des Applikationskopfs auf dem Auge aufliegt und das Auge deformiert. Der Schaltkreis ist eingerichtet, das dreidimensionale Bearbeitungsmodell in ein deformiertes dreidimensionales Bearbeitungsmodell eines im Auge zu bearbeitenden deformierten dreidimensionalen Bearbeitungsmusters zu transformieren, unter Verwendung der Referenzstrukturen einen im am Auge fixierten Zustand des Applikationskopfs verschobenen Positionierungsreferenzpunkt zu bestimmen, und das deformierte dreidimensionale Bearbeitungsmodell im am deformierten Auge fixierten Zustand des Applikationskopfs bezüglich des verschobenen Positionierungsreferenzpunkt zu positionieren, und das Scannersystem im am deformierten Auge fixierten Zustand des Applikationskopfs derart zu steuern, dass das Scannersystem den gepulsten Laserstrahl gemäss dem bezüglich des verschobenen Positionierungsreferenzpunkts positionierten deformierten dreidimensionalen Bearbeitungsmodell auf die Bearbeitungszielpunkte des im Auge zu bearbeitenden deformierten dreidimensionalen Bearbeitungsmusters lenkt.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt schematisch als Blockdiagramm eine mittels eines Patienteninterfaces an einem Auge fixierte ophthalmologische Vorrichtung mit Laserquelle und Fokussieroptik zum Bearbeiten des Auges.
- Figur 2:: zeigt schematisch als Blockdiagramm eine mittels des Patienteninterfaces verkippt am Auge fixierte ophthalmologische Vorrichtung mit Laserquelle und Fokussieroptik zum Bearbeiten des Auges.
- Figur 3:: zeigt ein Flussidaramm, welches eine beispielhafte Abfolge von Schritten zur Behandlung eines Auges illustriert.
- Figur 4:: zeigt schematisch im Querschnitt ein Auge mit Augenstrukturen, welche im Wesentlichen ringförmig um eine Zentrumsachse der Vorderkammer des Auges angeordnet sind, sowie ein im Auge zu bearbeitendes dreidimensionales Bearbeitungsmuster.
- Figur 5:: zeigt schematisch das Auge im Querschnitt in einem durch einen Kontaktkörper deformierten Zustand mit den ringförmig um die Zentrumsachse der Vorderkammer angeordneten Augenstrukturen und dem im Auge zu bearbeitenden dreidimensionalen Bearbeitungsmuster.
- Figur 6:: zeigt schematisch ein Modell des Auges im Querschnitt im deformierten und im nicht deformierten Zustand, mit den ringförmig um die Zentrumsachse der Vorderkammer angeordneten Augenstrukturen und einem dreidimensionalen Bearbeitungsmodell des im Auge zu bearbeitenden dreidimensionalen Bearbeitungsmusters.
- Figur 7:: zeigt schematisch das Augenmodell im Querschnitt mit einem Positionierungsreferenzpunkt zur Positionierung des dreidimensionalen Bearbeitungsmodells, welcher mittels Distanzangaben bezüglich der ringförmig um die Zentrumsachse der Vorderkammer angeordneten Augenstrukturen definiert ist.
- Figur 8:: zeigt schematisch das Auge im Querschnitt mit einer Ausrichtung, die bezüglich einer optischen Achse einer Fokussieroptik verkippt ist, und einem durch die Verkippung verschobenen Positionierungsreferenzpunkt , welcher durch die Distanzangaben bezüglich der ringförmig um die Zentrumsachse der Vorderkammer angeordneter Augenstrukturen definiert ist.
- Figur 9:: zeigt schematisch das Augenmodell im Querschnitt mit einem Positionierungsreferenzpunkt zur Positionierung des dreidimensionalen Bearbeitungsmodells, welcher bezüglich einer Referenzebene definiert ist, die durch ringförmig um die Zentrumsachse der Vorderkammer angeordnete Augenstrukturen verläuft.
- Figur 10:: zeigt schematisch das Auge im Querschnitt mit der Ausrichtung, die bezüglich der optischen Achse einer Fokussieroptik verkippt ist, und einem durch die Verkippung verschobenen Positionierungsreferenzpunkt, welcher bezüglich der Referenzeben definiert ist, die durch die ringförmig um die Zentrumsachse der Vorderkammer angeordneten Augenstrukturen verläuft.
- Figur 11:: zeigt schematisch das Augenmodell im Querschnitt mit einem Positionierungsreferenzpunkt zur Positionierung des dreidimensionalen Bearbeitungsmodells, welcher mittels Distanzstrecken bezüglich ringförmig um die Zentrumsachse der Vorderkammer angeordneter Augenstrukturen definiert ist.
- Figur 12:: zeigt schematisch das Augenmodell im Querschnitt in einem deformierten Zustand und einem durch die Deformation verschobenen Positionierungsreferenzpunkt, welcher mittels verkürzter Distanzstrecken bezüglich der ringförmig um die Zentrumsachse der Vorderkammer angeordneten Augenstrukturen definiert ist.
- Figur 13:: zeigt schematisch das Auge im Querschnitt im deformierten Zustand und mit der Ausrichtung, die bezüglich der optischen Achse einer Fokussieroptik verkippt ist, und einem durch die Deformation und Verkippung verschobenen Positionierungsreferenzpunkt, welcher durch Distanzangaben bezüglich der ringförmig um die Zentrumsachse der Vorderkammer angeordneten Augenstrukturen definiert ist.
- Figur 14:: zeigt schematisch das Augenmodell im Querschnitt mit einem Positionierungsreferenzpunkt zur Positionierung des dreidimensionalen Bearbeitungsmodells, welcher bezüglich einer Referenzebene definiert ist, die durch ringförmig um die Zentrumsachse der Vorderkammer angeordnete Augenstrukturen verläuft.
- Figur 15:: zeigt schematisch das Augenmodell im Querschnitt in einem deformierten Zustand und einem durch die Deformation verschobenen Positionierungsreferenzpunkt, welcher bezüglich der Referenzebene definiert ist, die durch die ringförmig um die Zentrumsachse der Vorderkammer angeordneten Augenstrukturen verläuft.
- Figur 16:: zeigt schematisch das Auge im Querschnitt im deformierten Zustand und mit der Ausrichtung, die bezüglich der optischen Achse einer Fokussieroptik verkippt ist, und einem durch die Deformation und Verkippung verschobenen Positionierungsreferenzpunkt, welcher bezüglich der Referenzebene definiert ist, die durch die ringförmig um die Zentrumsachse der Vorderkammer angeordneten Augenstrukturen verläuft.
- Figur 17:: zeigt schematisch im Querschnitt das Auge mit der bezüglich der optischen Achse einer Fokussieroptik verkippten Ausrichtung und einem dreidimensionalen Bearbeitungsmuster, das im Auge gemäss einem dreidimensionalen Bearbeitungsmodell zu bearbeiten ist, welches bezüglich der Referenzstrukturen ausgerichtet und bezüglich des durch die Verkippung verschobenen Positionierungsreferenzpunkts positioniert ist.
- Figur 18:: zeigt schematisch im Querschnitt das Auge, im deformierten Zustand und mit der bezüglich der optischen Achse einer Fokussieroptik verkippten Ausrichtung, und ein dreidimensionales Bearbeitungsmuster, das im Auge gemäss einem dreidimensionalen Bearbeitungsmodell zu bearbeiten ist, welches bezüglich der Referenzstrukturen ausgerichtet und bezüglich des durch die Deformation und Verkippung verschobenen Positionierungsreferenzpunkts positioniert ist.
- Figur 19:: zeigt schematisch das Augenmodell in der Aufsicht mit den ringförmig um die Zentrumsachse der Vorderkammer angeordneten Augenstrukturen mit zwei gegenüberliegenden Punkten und diesbezüglichen Abständen eines Positionierungsreferenzpunkts.
- Figur 20:: zeigt schematisch das Auge in der Aufsicht mit den ringförmig um die Zentrumsachse der Vorderkammer angeordneten Augenstrukturen mit zwei gegenüberliegenden Punkten und diesbezüglichen Abständen eines Positionierungsreferenzpunkts.
- Figur 21:: zeigt schematisch das Augenmodell in der Aufsicht mit den ringförmig um die Zentrumsachse der Vorderkammer angeordneten Augenstrukturen mit drei darauf liegenden Punkten und diesbezüglichen Abständen eines Positionierungsreferenzpunkts.
- Figur 22:: zeigt schematisch das Auge in der Aufsicht mit den ringförmig um die Zentrumsachse der Vorderkammer angeordneten Augenstrukturen mit drei darauf liegenden Punkten und diesbezüglichen Abständen eines Positionierungsreferenzpunkts.
- Figur 23:: zeigt schematisch das Augenmodell in der Aufsicht mit den ringförmig um die Zentrumsachse der Vorderkammer angeordneten Augenstrukturen mit vier darauf liegenden Punkten und diesbezüglichen Abständen eines Positionierungsreferenzpunkts.
- Figur 24:: zeigt schematisch das Auge in der Aufsicht mit den ringförmig um die Zentrumsachse der Vorderkammer angeordneten Augenstrukturen mit vier darauf liegenden Punkten und diesbezüglichen Abständen eines Positionierungsreferenzpunkts.

### Wege zur Ausführung der Erfindung

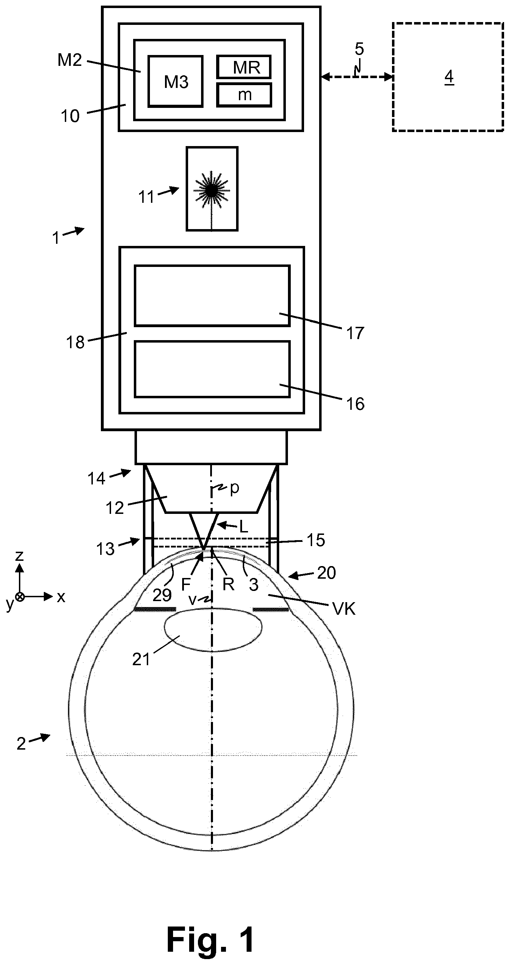

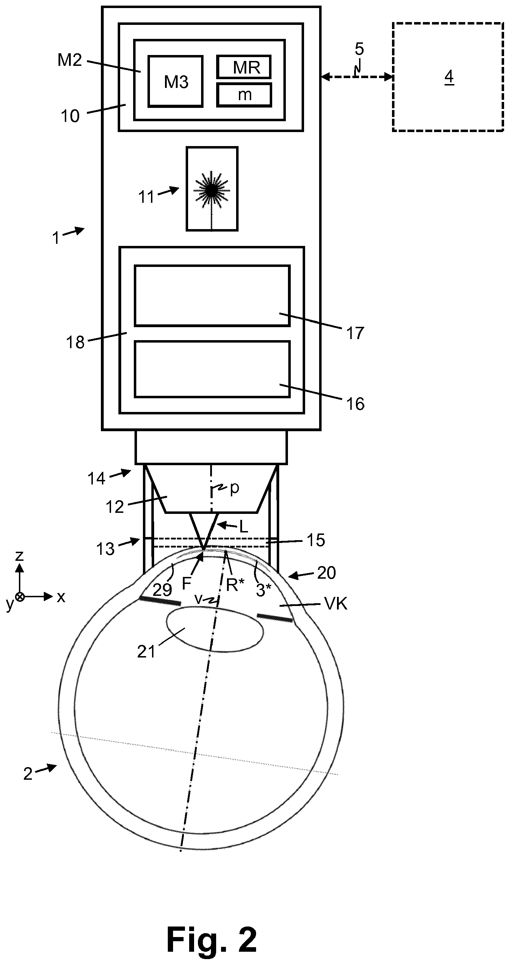

In den Figuren 1 und 2 bezieht sich das Bezugszeichen 1 auf eine ophthalmologische Vorrichtung zum Bearbeiten eines Auges 2 mittels eines gepulsten Laserstrahls L. Die ophthalmologische Vorrichtung 1 umfasst eine Laserquelle 11, die eingerichtet ist, den gepulsten Laserstrahl L zu erzeugen, beispielsweise ein mit Femtosekundenlaserpulsen gepulster Laserstrahl L.

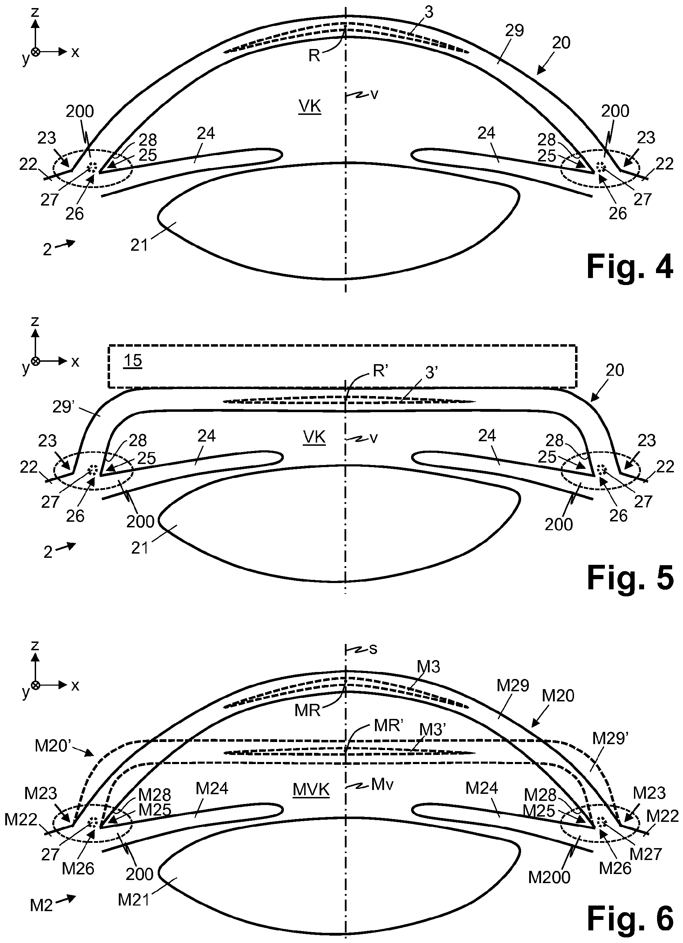

Wie in den Figuren 1 und 2 schematisch dargestellt ist, umfasst die ophthalmologische Vorrichtung 1 einen Applikationskopf 14 mit einer Fokussieroptik 12 und einem Patienteninterface 13. Das Patienteninterface 13 ist eingerichtet, den Applikationskopf 14 am Auge 2 zu fixieren. Das Patienteninterface 13 umfasst dazu beispielsweise einen Saugring um den Applikationskopf 14 mittels Unterdruck am Auge 2 zu fixieren. In einer Ausführungsvariante ist das Patienteninterface 13 als flüssigkeitsbasiertes Patienteninterface 13 ausgeführt, bei welchem im angedockten Zustand das Auge 2 eine Interfacekammer zwischen der Fokussieroptik 12 und dem angedockten Auge 2 erzeugt wird, die über eine Zuführleitung mit einer lichtdurchlässigen Flüssigkeit gefüllt wird. In einer Ausführungsvariante umfasst das Patienteninterface 13 einen optionalen Kontaktkörper 15, welcher im angedockten Zustand das Auge 2 deformiert, beispielsweise applaniert oder in eine andere vorgegebene Form des Kontaktkörpers 15 führt, wie beispielsweise in den Figuren 5, 13, 16, 18 illustriert ist.

Der gepulste Laserstrahl L wird von der Laserquelle 11 über ein optisches Übertragungssystem 18 der Fokussieroptik 12 zugeführt. Die Fokussieroptik 12 weist eine optische Achse p auf und umfasst ein Linsensystem mit mehreren optischen Linsen und ist eingerichtet, den gepulsten Laserstrahl L im am Auge 2 angedockten Zustand des Applikationskopf 14 im Auge 2 zu fokussieren, in Augengewebe 20, beispielsweise in die Cornea 29 oder in die Augenlinse 21, oder in ein im Auge angeordnetes Implantat. Die Laserquelle 11 und der Applikationskopf 14 sind beispielsweise in separaten Gehäusen angeordnet, die über einen starren oder beweglichen Tragarm miteinander verbunden sind.

In der Figur 1 ist die ophthalmologische Vorrichtung 1 im am Auge 2 angedockten Zustand dargestellt, wobei die optische Achse p der Fokussieroptik 12 koaxial zur Zentrumsachse v der Vorderkammer VK des angedockten Auges 2 ausgerichtet ist.

In der Figur 2 ist die ophthalmologische Vorrichtung 1 im am Auge 2 angedockten Zustand dargestellt, wobei das Auge 2 zum Applikationskopf 14 verkippt ist, so dass die Zentrumsachse v der Vorderkammer VK des angedockten Auges 2 verkippt zur optischen Achse p der Fokussieroptik 12 ausgerichtet ist (d.h. die optische Achse p der Fokussieroptik 12 und die Zentrumsachse v der Vorderkammer VK verlaufen weder koaxial noch parallel zueinander).

Das optische Übertragungssystem 18 umfasst ein Scannersystem 17, das eingerichtet ist, den gepulsten Laserstrahl L gemäss einem dreidimensionalen (x, y, z) Bearbeitungsmodell M3 auf Bearbeitungszielpunkte F eines im Auge 2 zu bearbeitenden dreidimensionalen Bearbeitungsmusters 3 zu lenken (gezielte Positionierung des Fokus des gepulsten Laserstrahls L auf x/y/z-Bearbeitungszielpunkte F im Auge 2 gemäss dem Bearbeitungsmodell M3). Abhängig von der Ausführungsvariante respektive dem Anwendungsmodus und den damit einhergehenden Parametern des gepulsten Laserstrahls L (insbesondere dessen Laserenergie und fokussierte Spotgrösse) definiert das Bearbeitungsmodell M3 ein Schnittmodell M3 eines im Augengewebe 20 zu schneidenden dreidimensionalen Schnittmusters 3, bei welchem Augengewebe zum Schneiden oder Abtragen aufgelöst respektive abgebaut wird, beispielsweise ein Lentikel, oder ein Bearbeitungsmodell M3 eines im Auge 2 zu bestrahlenden dreidimensionalen Bearbeitungsmusters 3, bei welchem das Augengewebe 20 oder ein Objekt im Auge 2, z.B. ein Implantat, verändert wird, beispielsweise hinsichtlich seiner optischen Eigenschaften wie Brechungsindex und/oder Lichtdurchlässigkeit, ohne dass es dabei aufgelöst oder abgebaut wird. Das Scannersystem 17 umfasst dazu ein oder mehrere strahlablenkende optische Elemente, beispielsweise steuerbare bewegliche Spiegel (x/y-Bearbeitungsrichtung) und Divergenzmodulatoren (z-Bearbeitungsrichtung), respektive steuerbare Linsensysteme, um den gepulsten Laserstrahl L auf die Bearbeitungszielpunkte F des dreidimensionalen Bearbeitungsmusters 3 zu lenken. In einer Ausführungsvariante umfasst das Scannersystem 17 ein Antriebssystem mit elektrischen Motoren zum Verfahren der Fokussieroptik 12 in einer oder mehreren Scann- respektive Bearbeitungsrichtungen.

Die ophthalmologische Vorrichtung 1 umfasst zudem ein Messsystem 16, das eingerichtet ist, im am Auge 2 angedockten Zustand (in-situ) Strukturen des Auges 2 optisch zu erfassen und zur weiteren Auswertung digitalisiert zu speichern. In einer Ausführungsvariante ist das Messsystem 16 eingerichtet, im am Auge 2 angedockten Zustand dreidimensionale Augenstrukturen zu erfassen. In einer Ausführungsvariante ist das Messsystem 16 eingerichtet, im am Auge 2 angedockten Zustand ein dreidimensionales (in-situ) Augenmodell zu erfassen. Das Messsystem 16 ist beispielsweise als interferometrisches Messsystem (für optische Kohärenztomografie, OCT), triangulierendes oder konfokales Messsystem ausgebildet, und/oder umfasst ein optisches Aufzeichnungsgerät zur Erfassung von Querschnittsbildern des Auges 2. Das interferometrische Messsystem ist eingerichtet, die Strukturen des Auges 2 über einen in Richtung einer optischen Achse p der Fokussieroptik 12 verlaufenden Tiefenbereich optisch zu erfassen, und ermöglicht die Erfassung dreidimensionaler Augenstrukturen (und Referenzstrukturen) des Auges 2 für ein dreidimensionales (in-situ) Augenmodell. Das interferometrische Messsystem ist in den optischen Pfad zur Fokussieroptik 12 eingekoppelt, beispielsweise über ein optisches Einkopplungselement, z.B. ein Strahlteilerwürfel oder ein Einkopplungsspiegel, in den optischen Pfad des optischen Übertragungssystems 18. Bei der Erfassung von Querschnittsbildern, beispielsweise durch Erfassen von beleuchteten Lichtquerschnitten im Auge 2 mittels eines oder mehreren in Scheimpflug angeordneten Bildsensoren, wie z.B. in EP 1358839 beschrieben, können aus mehreren erfassten Querschnittsbildern von beleuchteten Lichtquerschnitten an verschiedenen Positionen, dreidimensionale Augenstrukturen respektive ein dreidimensionales (in-situ) Augenmodel erfasst werden, wie z.B. in EP 1430829 beschrieben wird.

Zur Steuerung der ophthalmologischen Vorrichtung 1 ist ein Schaltkreis 10 vorgesehen, der über Steuer- respektive Datenleitungen mit der Laserquelle 11, dem Scannersystem 17 und dem Messsystem 16 verbunden ist. Der Schaltkreis 10 umfasst beispielsweise ASICs (Application Specific Integrated Circuits), durch Programmcode programmierte Mikroprozessoren und/oder andere elektronische Schaltungen. Wie in den Figuren 1 und 2 schematisch dargestellt ist, umfasst der Schaltkreis 10 Datenspeicher zur Speicherung eines dreidimensionalen Augenmodells M2 und eines dreidimensionalen Bearbeitungsmodells M3 eines im Auge 2 zu bearbeitenden dreidimensionalen Bearbeitungsmusters 3 mit einem Positionierungsreferenzpunkt MR und Referenzmessdaten m zur Positionierung des Bearbeitungsmodells M3 im Auge 2. Der Schaltkreis 10 ist beispielsweise eingerichtet, das Augenmodell M2, einschliesslich der nachfolgend näher beschriebenen (dreidimensionalen) Augenstrukturen, und das dreidimensionale Bearbeitungsmodell M3 mit den Positionierungsdaten (Positionierungsreferenzpunkt MR, Referenzmessdaten m) über eine Kommunikationsstrecke 5, z.B. über eine LAN oder WLAN Verbindung ((Wireless) Local Area Network), von einer externen Planungsvorrichtung 4 zu empfangen.

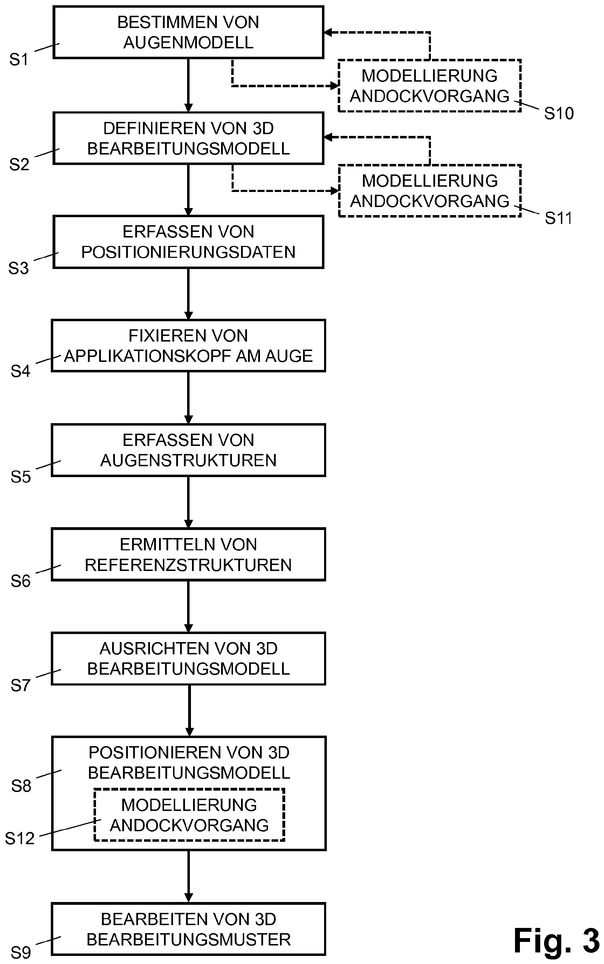

Nachfolgend wird mit Bezug zur Figur 3 eine Abfolge von Schritten zur Behandlung des Auges 2 beschrieben.

In einem vorbereitenden Schritt S1 wird mittels einer externen ophthalmologischen Planungsvorrichtung 4 ein dreidimensionales Augenmodell M2 des zu behandelnden Auges 2 erfasst und abgespeichert. Dabei werden die Augenstrukturen des Auges 2 bestimmt und als Teil des Augenmodells M2 gespeichert, wie nachfolgend erläutert wird. Geeignete Planungsvorrichtungen 4 sind dem Fachmann bekannt. Die Ermittlung des Augenmodells M2 erfolgt im bestimmt ausgerichteten Zustand des Auges 2, beispielsweise durch Vorgabe eines Blickrichtungsziels, auf das der Patient seinen Blick während des Messvorgangs ausrichtet, oder umfasst bei einer Erfassung ohne Blickrichtungsfixierung eine anschliessende Ausrichtung des Augenmodells M2. In beiden Fällen wird die Zentrumsachse v der Vorderkammer VK des Auges 2 beispielsweise koaxial zur optischen Achse der Messoptik respektive zu einer vertikalen Referenzachse der Planungsvorrichtung 4 ausgerichtet (beispielsweise in z-Richtung). Die an die Erfassung anschliessende Ausrichtung des Augenmodells M2 erfolgt beispielsweise durch einen Benutzer mittels geeigneter Softwareanwendungen in der externen Planungsvorrichtung 4, z.B. mittels computergestützter Design Programme (CAD-Anwendungen), welche dem Fachmann bekannt sind, oder die Zentrumsachse v der Vorderkammer VK des Auges 2 wird durch ein funktionales Zusatzmodul in der externen Planungsvorrichtung 4 basierend auf den ermittelten Augenstrukturen bestimmt, insbesondere aufgrund von ringförmig um die Zentrumsachse v angeordneten (dreidimensionale) Referenzstrukturen 200, wie nachfolgend im Zusammenhang mit Schritt S7 zur Ausrichtung eines Bearbeitungsmodells beschrieben wird, und als vertikale Referenzachse der Planungsvorrichtung 4 verwendet respektive zur vertikalen Referenzachse der Planungsvorrichtung 4 ausgerichtet.

Figur 4 illustriert das zu behandelnde Auge 2 und das im Auge 2 zu bearbeitende dreidimensionale Bearbeitungsmuster 3 schematisch im Querschnitt. In der Figur 4 sind neben der Sklera 22 die Cornea 29 und die Augenlinse 21 ersichtlich, welche die Ausdehnung der Vorderkammer VK des Auges 2 definieren, welche auch als vordere Augenkammer oder "Camera anterior bulbi" bezeichnet wird. In der Figur 4 ist zudem die Zentrumsachse v der Vorderkammer VK dargestellt. Abhängig von der Definition reicht die Vorderkammer VK des Auges 2 zumindest von der hinteren Fläche 29p der Cornea 29 (Hornhaut) bis zur Iris 24 (Regenbogenhaut). Im vorliegenden Kontext wird auch der Bereich bis zur Augenlinse 21 als Teil der Vorderkammer VK betrachtet.

Figur 6 illustriert schematisch im Querschnitt ein Augenmodell M2 des in Figur 4 dargestellten Auges 2. Die Sklera 22, die Cornea 29, die Augenlinse 21 und die dadurch definierte Vorderkammer VK des Auges 2 sind im Augenmodell M2 als modellierte Augenstrukturen Sklera M22, Cornea M29, Augenlinse M21 und Vorderkammer MVK wiedergegeben. Die Zentrumsachse v der Vorderkammer VK ist im Augenmodell M2 der Figur 6 mit Mv bezeichnet.

Des Weiteren sind in der schematischen Querschnittsdarstellung des Auges 2 der Figur 4 Referenzstrukturen 200 dargestellt, die im Wesentlichen ringförmig (und konzentrisch) um die Zentrumsachse v der Vorderkammer VK angeordnet sind. Diese ringförmigen Referenzstrukturen 200 der Vorderkammer VK respektive um die Vorderkammer VK sind für die vorliegende ophthalmologischen Vorrichtung 1 und das damit unter Steuerung durch den Schaltkreis 10 ausgeführte Verfahren von besonderer Bedeutung. Diese Referenzstrukturen 200 umfassen den Limbus 23, die Iris 24, den Vorderkammerwinkel 25, den Skleral Spur 26, den Schlemmschen Kanal 27 und/oder die Schwalbes Linie 28. Im Augenmodell M2 der Figur 6 sind die entsprechenden modellierten Referenzstrukturen in der modellierten Struktur der Vorderkammer MVK mit dem Bezugszeichen M200 versehen und umfassen die modellierten Augenstrukturen Limbus M23, Iris M24, Vorderkammerwinkel M25, Skleral Spur M26, Schlemmschen Kanal M27 und/oder Schwalbes Linie M28.

Figur 5 illustriert schematisch im Querschnitt das zu behandelnde Auge 2 in einem mittels des Patienteninterfaces 13 an den Applikationskopf 14 angedockten Zustand, in welchem das Auge 2 durch einen applizierten Kontaktkörper 15 des Patienteninterfaces 13 deformiert ist. Wie in der schematischen Darstellung ersichtlich ist, ist dabei vorwiegend die Cornea 29 deformiert, wohingegen die ringförmige um die Zentrumsachse v der Vorderkammer VK angeordneten Referenzstrukturen 200 im Wesentlichen unverändert bleiben. Mit anderen Worten, der Limbus 23, die Iris 24, der Vorderkammerwinkel 25, der Sklerale Spur 26, der Schlemmsche Kanal 27 und die Schwalbes Linie 28 werden durch den applizierten Kontaktkörper 15 nicht wesentlich deformiert.

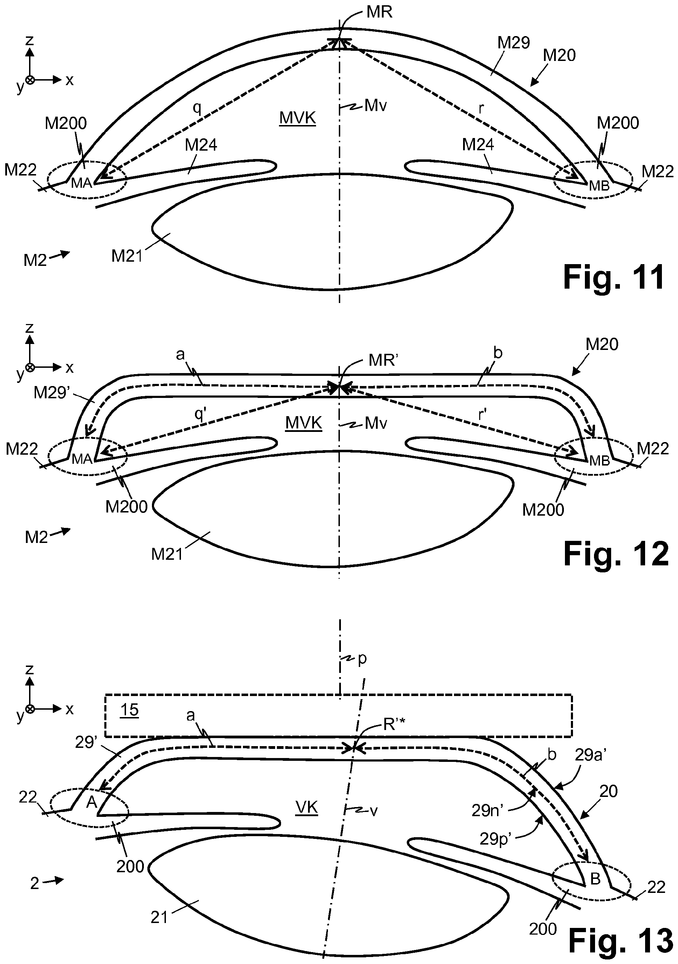

In der Figur 6 ist im schematisch dargestellten Querschnitt des Augenmodells M2 auch das Modell des in Figur 5 dargestellten deformierten Auges 2 illustriert, dabei ist das modellierte deformierte Augengewebe M20' respektive das Modell der deformierten Cornea M29' dargestellt. Abhängig von der Ausführungsvariante, wird im optionalen Schritt S10 der Andockvorgang des Applikationskopf 14 mittels dessen Patienteninterface 13 an das zu behandelnde Auge 2 modelliert und ein Augenmodell M2 im angedockten Zustand erzeugt. Gegebenenfalls wird dabei der Kontaktkörper 15 und die durch dessen Applikation auf dem Auge 2 bewirkte Deformation durch ein funktionales Zusatzmodul in der externen Planungsvorrichtung 4 modelliert und das Augenmodell M2 des deformierten Auges 2, insbesondere das Modell der deformierten Cornea M29', aufgrund des nicht deformierten dreidimensionalen Augenmodells M2 erzeugt. In einer Ausführungsvariante werden für verschiedene Verkippungsgrade des Auges 2, beispielsweise hinsichtlich der Zentrumsachse v seiner Vorderkammer VK bezüglich einer optischen Achse p der Fokussieroptik 12, wie in den Figuren 13, 16 und 18 illustriert ist, jeweils unterschiedliche Varianten des Augenmodells M2 des deformierten und verkippten Auges 2, insbesondere der deformierten und verkippten Cornea M29', erzeugt und gespeichert. Dabei ist das funktionale Zusatzmodul ein programmiertes Softwaremodul, das einen Prozessor der externen Planungsvorrichtung 4 (oder des Schaltkreises 10) derart steuert, dass dieser die angeführten Zusatzfunktionen in der externen Planungsvorrichtung 4 (respektive im Schaltkreis 10) ausführt.

Auf der Basis des erzeugten (deformierten oder nicht deformierten) Augenmodells M2 wird im Schritt S2 das dreidimensionale Bearbeitungsmodell M3 des im Auge 2 zu bearbeitenden dreidimensionalen Bearbeitungsmusters 3 definiert und gespeichert. Dies wird durch einen Benutzer mittels geeigneter Softwareanwendungen in der externen Planungsvorrichtung 4 durchgeführt, z.B. im Augenmodell M2 mittels computergestützter Design Programme (CAD-Anwendungen), welche dem Fachmann bekannt sind, und ist durch die gewünschte Behandlung des Auges 2 bestimmt, beispielsweise eine bestimmte refraktive Korrektur des Auges 2 oder ein anderer bearbeitender Eingriff im Augengewebe 20 oder an einem im Auge 2 angeordneten Objekt (Implantat) mittels des gepulsten Laserstrahls L.

Im Beispiel der Figur 4 ist das im Auge 2 zu bearbeitende Bearbeitungsmuster 3 schematisch im Querschnitt mit gestrichelter Linie als dreidimensionales Bearbeitungsmuster dargestellt, z.B. ein Schnittmuster für ein dreidimensionales Gewebevolumen wie ein Lentikel oder eine andere Form, das in der Cornea 29 zu schneiden ist. In der Figur 4 ist zudem ein Positionierungsreferenzpunkt R des im Auge 2 zu bearbeitenden Bearbeitungsmusters 3 dargestellt, welcher nachfolgend im Zusammenhang mit dem dreidimensionalen Bearbeitungsmodell M3 des zu bearbeitenden Bearbeitungsmusters 3 näher erläutert wird. Das entsprechende dreidimensionale Bearbeitungsmodell M3 ist im Augenmodell M2 der Figur 6 ebenfalls mit gestrichelter Linie innerhalb der modellierten Augenstruktur der Cornea M29 wiedergegeben. Wie in der Figur 6 schematisch dargestellt ist, verläuft die Zentrumsachse s des dreidimensionalen Bearbeitungsmodells M3 im Augenmodell M2 parallel zur Zentrumsachse Mv der modellierten Vorderkammer MVK.

Figur 5 illustriert schematisch auch das im Auge 2 zu bearbeitende dreidimensionale Bearbeitungsmuster 3' im entsprechend deformierten Zustand. Das entsprechende dreidimensionale deformierte Bearbeitungsmodell M3' ist im Augenmodell M2 der Figur 6 ebenfalls mit gestrichelter Linie innerhalb der modellierten Augenstruktur der deformierten Cornea M29' wiedergegeben. Abhängig von der Ausführungsvariante definiert der Benutzer bei einer geplanten Anwendung des Kontaktkörpers 15 das Bearbeitungsmodell M3 des zu bearbeitenden Bearbeitungsmuster 3 im nicht deformierten Zustand des Auges 2 respektive des Augenmodells M2, und das funktionale Zusatzmodul der externen Planungsvorrichtung 4 (respektive des Schaltkreises 10) erzeugt im optionalen Schritt S11 ein deformiertes Bearbeitungsmodell M3', oder der Benutzer definiert das deformierte Bearbeitungsmodell M3' im Augenmodell M2 des Auges 2 im deformierten Zustand, im Modell der deformierten Augengewebes M20' insbesondere im Modell der deformierten Cornea M29'. Im optionalen Schritt S11 wird somit der Andockvorgang des Applikationskopf 14 mittels dessen Patienteninterface 13 an das zu behandelnde Auge 2 hinsichtlich dessen Einflusses auf das Bearbeitungsmodell M3 modelliert und ein Bearbeitungsmodell M3' im angedockten Zustand erzeugt. In einer Ausführungsvariante werden für verschiedene Verkippungsgrade des Auges 2, beispielsweise hinsichtlich der Zentrumsachse v seiner Vorderkammer VK bezüglich der optischen Achse p der Fokussieroptik 12, jeweils unterschiedliche Varianten des Bearbeitungsmodells M3' im angedockten Zustand erzeugt und gespeichert.

Im Schritt S3 werden Positionierungsdaten zur Positionierung des dreidimensionalen Bearbeitungsmodells M3 (oder des erzeugten deformierten Bearbeitungsmodells M3') bestimmt und gespeichert. Dies erfolgt automatisiert und/oder benutzerdefiniert mittels eines programmierten Softwaremoduls eines funktionalen Zusatzmoduls, das auf einem Prozessor der externen Planungsvorrichtung 4 oder eines anderen Computers ausgeführt wird. Hierfür wird zunächst ein Positionierungsreferenzpunkt MR zur Positionierung des dreidimensionalen Bearbeitungsmodells M3 (oder ein Positionierungsreferenzpunkt MR' zur Positionierung des deformierten Bearbeitungsmodells M3') im Auge 2 respektive im Augenmodell M2 definiert. Der Positionierungsreferenzpunkt MR (MR') ist beispielsweise ein automatisch gewählter Zentrumspunkt oder "Schwerpunkt" im definierten Bearbeitungsmodell M3 (oder im deformierten Bearbeitungsmodell M3') oder ein ausgewählter, mittels Mauszeiger (Cursor) und/oder anderen Bedienelementen benutzerdefinierter Punkt im definierten Bearbeitungsmodell M3 (oder im deformierten Bearbeitungsmodell M3'). Der Positionierungsreferenzpunkt MR für das definierte Bearbeitungsmodell M3 ist beispielsweise in der Figur 6 dargestellt. Figur 6 illustriert zudem einen Positionierungsreferenzpunkt MR' für das deformierte Bearbeitungsmodell M3'. An dieser Stelle soll angeführt werden, dass der Positionierungsreferenzpunkt MR, MR' nicht auf der Zentrumsachse v, Mv der Vorderkammer VK, MVK liegen muss, wie dies in den Figuren dargestellt ist, sondern vom Benutzer respektive vom funktionalen Zusatzmoduls auch an anderen Positionen im Bearbeitungsmodell M3, M3' respektive im Augenmodell M2 definiert werden kann.

Als nächstes werden für den definierten Positionierungsreferenzpunkt MR, MR' Referenzmessdaten m zur späteren Positionierung des Positionierungsreferenzpunkts MR, MR' bezüglich der Referenzstrukturen 200 im Auge 2 ermittelt. Dazu werden die Referenzmessdaten m für den Positionierungsreferenzpunkt MR, MR' im Augenmodell M2 bezüglich der modellierten Referenzstrukturen M200 der modellierten Vorderkammer MVK bestimmt, insbesondere bezüglich der modellierten Augenstrukturen Limbus M23, Iris M24, Vorderkammerwinkel M25, Skleral Spur M26, Schlemmschen Kanal M27 und/oder Schwalbes Linie M28.

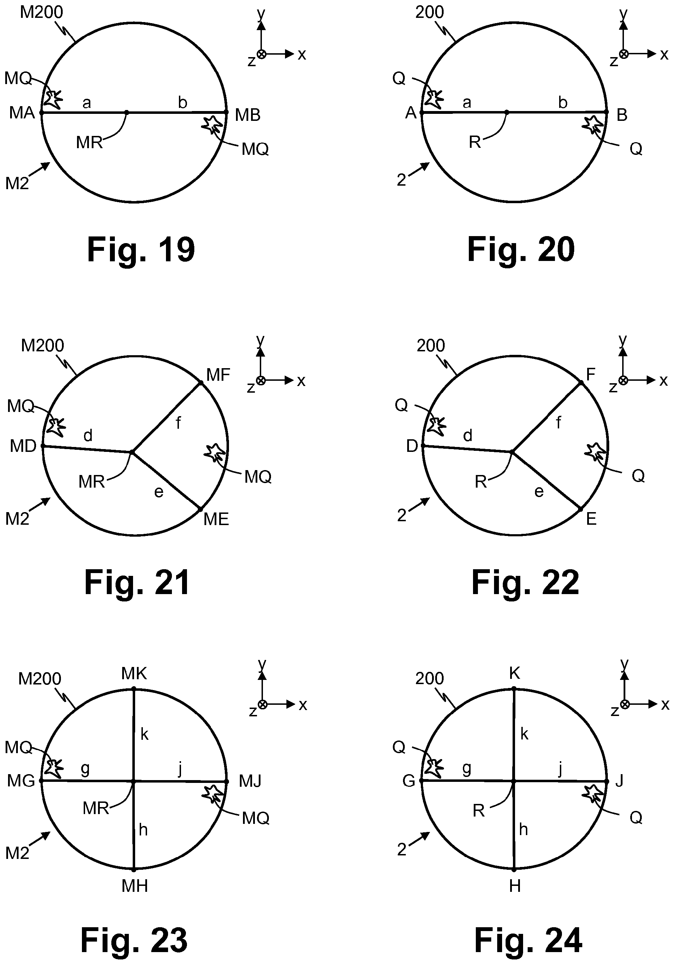

In einer ersten Ausführungsvariante werden als Referenzmessdaten m Distanzgaben vom Positionierungsreferenzpunkt MR, MR' im Augenmodell M2 bezüglich ausgewählter Bezugspunkte auf den modellierten Referenzstrukturen M200 der modellierten Vorderkammer MVK verwendet. Wie in der Aufsicht des Augenmodells M2 in der Figur 19 ersichtlich ist, werden dazu im Augenmodell M2 Bezugspunkte MA, MB gewählt, die auf den modellierten ringförmigen Referenzstrukturen M200 einander gegenüberliegen und die Distanzen a, b vom Positionierungsreferenzpunkt MR, MR' im Augenmodell M2 zu diesen Bezugspunkten werden als Referenzmessdaten m erfasst (ermittelt und gespeichert). Die Lage der Bezugspunkte MA, MB auf den ringförmigen Referenzstrukturen M200 im Augenmodell M2 wird dabei beispielsweise mittels Polarkoordinaten bezüglich der Zentrumsachse Mv der Vorderkammer MKV definiert, unter Verwendung von strukturellen und/oder geometrisch bestimmbaren Merkmalen oder Muster MQ im Augengewebe 20 zur Bestimmung der Verdrehung um die Zentrumsachse Mv der Vorderkammer MKV, beispielsweise Merkmale und/oder Muster der Iris M24 aus einem Aufsichtsbild des Auges 2. In alternativen Ausführungsvarianten der Figuren 21 und 23 werden im Augenmodell M2 mehrere verteilte (z.B. drittelrespektive viertelsweise) Bezugspunkte MD, ME, MF, MG, MH, MJ, MK auf den modellierten ringförmigen Referenzstrukturen M200 gewählt und die Distanzen d, e, f, g, h, j respektive k vom Positionierungsreferenzpunkt MR, MR' im Augenmodell M2 zu diesen Bezugspunkten MD, ME, MF, MG, MH, MJ, MK als Referenzmessdaten m erfasst. Die Bezugspunkte MA, MB, MC, MD, ME, MF, MG, MH, MJ, MK liegen somit entsprechend auf den modellierten Augenstrukturen Limbus M23, Iris M24, Vorderkammerwinkel M25, Skleral Spur M26, Schlemmschen Kanal M27 respektive Schwalbes Linie M28.

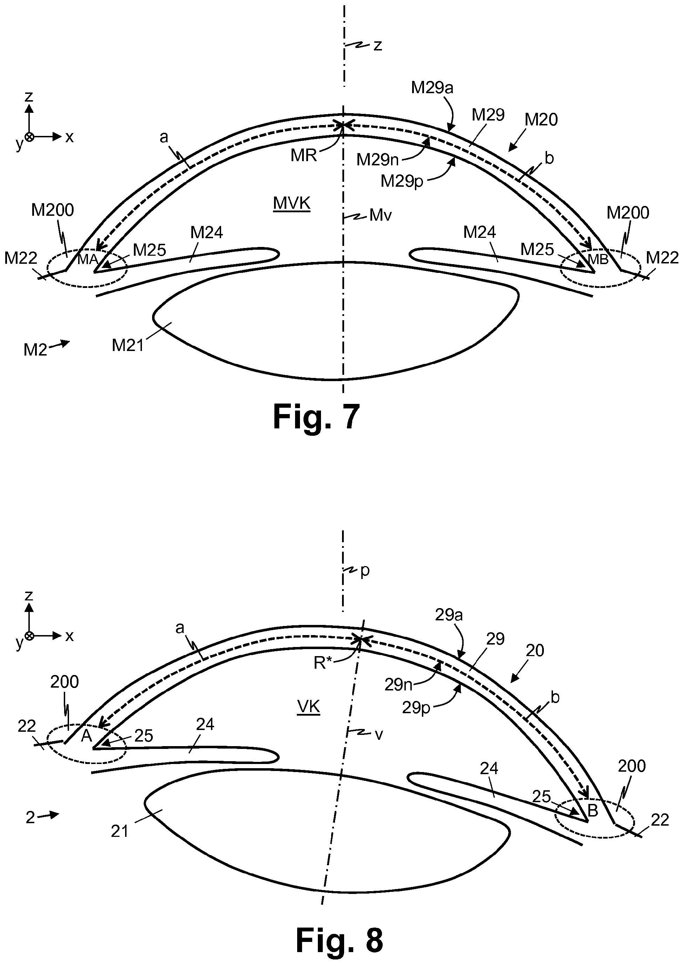

In der Figur 7 sind im Querschnitt des Augenmodells M2 die Distanzen a, b des Positionierungsreferenzpunkt MR schematisch bezüglich der zwei gegenüberliegenden Bezugspunkten MA, MB auf den modellierten ringförmigen Referenzstrukturen M200 dargestellt. Auch wenn dies hier nicht weiter beschrieben oder illustriert wird, gelten die Ausführungen entsprechend auch für mehrere Bezugspunkte MA, MB, MC, MD, ME, MF, MG, MH, MJ, MK. Gemäss der Ausführungsvariante der Figur 7 entsprechen die Distanzen a, b Wegstrecken, die entlang von modellierten Augenstrukturen des Augenmodells M2 verlaufen. Im Beispiel der Figur 7 entsprechen die Distanzen a, b Wegstrecken, die auf einer neutralen Faser M29n in der Cornea M29 verlaufen (die neutrale Faser der Cornea ist deshalb besonders vorteilhaft, weil sich ihre Länge auch bei einer Deformation der Cornea durch einen applizierten Kontaktkörper 15 nicht verändert). In weiteren Ausführungsvarianten entsprechen die Distanzen a, b Wegstrecken die entlang der modellierten äusseren (vorderen) Oberfläche der Cornea 29a oder entlang der modellierten inneren (hinteren) Oberfläche der Cornea 29p verlaufen. In einer weiteren Ausführungsvariante werden die Distanzen q, r als direkte, kürzeste Strecken zwischen dem Positionierungsreferenzpunkt MR und den Bezugspunkten MA, MB auf den modellierten ringförmigen Referenzstrukturen M200 bestimmt, wie dies im Beispiel der Figur 11 illustriert wird.

In der Figur 12 ist die erste Ausführungsvariante angewendet auf das Augenmodell M2 im deformierten Zustand des Auges 2 schematisch im Querschnitt illustriert. In der Figur 12 sind die Wegstrecken a, b des Positionierungsreferenzpunkt MR' (entlang der neutralen Faser 29n der Cornea 29) im Augenmodell M2 des deformierten Zustands des Auges 2 schematisch bezüglich zwei gegenüberliegenden Bezugspunkten MA, MB auf den modellierten ringförmigen Referenzstrukturen M200 dargestellt. In der Figur 12 sind überdies (alternativ) die direkten Distanzen q', r' des Positionierungsreferenzpunkt MR' im Augenmodell M2 des deformierten Zustands des Auges 2 schematisch bezüglich der beiden gegenüberliegenden Bezugspunkte MA, MB dargestellt. An dieser Stelle soll angeführt werden, dass das Verhältnis q:r der direkten Distanzen q, r im Augenmodell M2 der Figur 11 in erster Näherung dem Verhältnis q':r' der direkten Distanzen q', r' im Augenmodell M2 des deformierten Zustands des Auges 2 der Figur 12 entspricht. Je geringer die Deformation, desto besser ist die Näherung. Daher ist die Verwendung der neutralen Faser 29n besonders vorteilhaft, da dort keine Deformation vorliegt. Tatsächlich entsprechen die Wegstrecken a, b entlang der neutralen Faser 29n der Cornea 29 im Augenmodell M2 der Figur 7 den Wegstrecken a, b entlang der neutralen Faser 29n der Cornea 29 im Augenmodell M2 des deformierten Zustands des Auges 2 der Figur 12. Weiterhin sind die (äussere) Vorderfläche 29a und die (innere) Rückfläche 29p der Cornea 29 vorteilhaft, da sie einerseits sehr gut vermessbar sind und andererseits deren Dehnung entlang der Oberflächen aufgrund der Steifigkeit der Cornea 29 sehr gering ist. Das Verhältnis bleibt unabhängig von der Deformation gleich. Die durch die Deformation verkürzten Distanzen q', r' werden entweder im deformierten Augenmodell M2 ermittelt oder, wenn die Deformation bekannt ist oder berechnet vorliegt, aufgrund der durch den Kontaktkörper 15 bewirkten Deformation aus den im Augenmodell M2 des Auges 2 im nicht deformierten Zustand ermittelten Distanzen q, r berechnet. Auch hier gelten die Ausführungen, wie obenstehend erwähnt, entsprechend für mehrere Bezugspunkte MA, MB, MC, MD, ME, MF, MG, MH, MJ, MK.

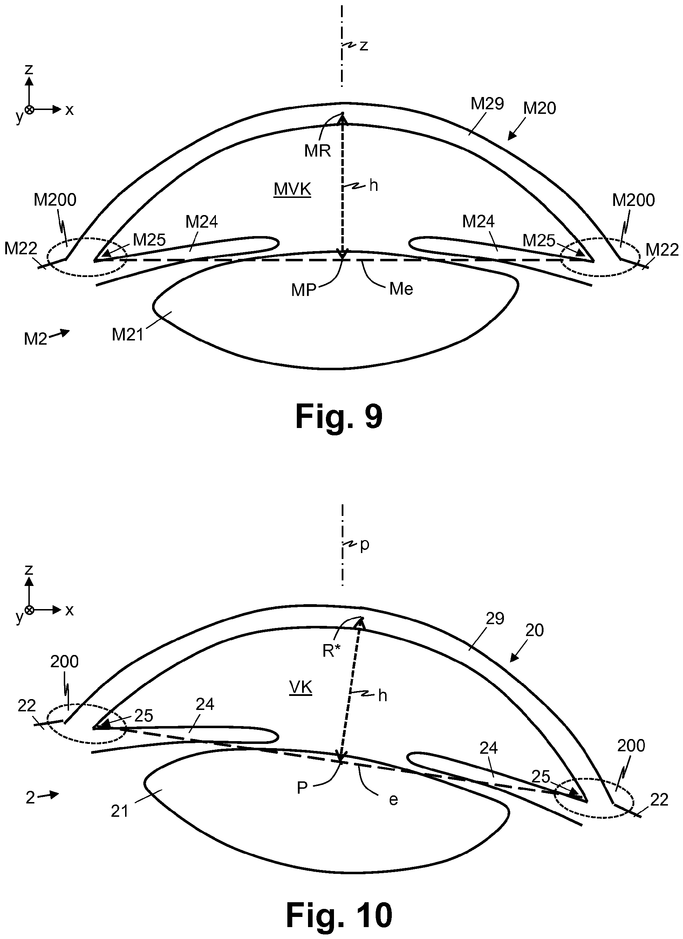

In der Figur 9 ist eine zweite Ausführungsvariante zur Erfassung von Referenzmessdaten m illustriert, in welcher eine Referenzebene Me bestimmt wird, welche durch die modellierten ringförmigen Referenzstrukturen M200 der modellierten Vorderkammer MVK definiert ist. Die Referenzebene Me verläuft beispielsweise durch die modellierten Augenstrukturen Limbus M23, Iris M24, Vorderkammerwinkel M25, Skleral Spur M26, Schlemmscher Kanal M27 oder Schwalbes Linie M28. Als Referenzmessdaten m werden eine Distanzangabe h und ein Projektionspunkt MP des Positionierungsreferenzpunkts MR bezüglich der Referenzebene Me ermittelt und gespeichert (eine Normale auf der Referenzebene Me durch den Projektionspunkt Mp verläuft durch den Positionierungsreferenzpunkt MR). Der Projektionspunkt MP des Positionierungsreferenzpunkts MR auf der Referenzebene Me wird bei der Planung bezüglich struktureller und/oder geometrisch bestimmbarer Merkmalen oder Muster MQ festgelegt, beispielsweise als Mittelpunkt mehrerer Referenzstrukturen auf der Referenzebene Me. Der Positionierungsreferenzpunkts MR wird dann im dreidimensionalen Bearbeitungsmodell M3 auf der durch den festgelegten Projektionspunkt MP verlaufenden Normale auf der Referenzebene Me bestimmt und mittels der Distanzangabe h zum Projektionspunkt MP definiert. Der Fachmann wird verstehen, dass der Positionierungsreferenzpunkts MR in einer alternativen Variante mittels eines zusätzlichen Offsetwerts beabstandet zu dieser Normalen definiert werden kann. Der Projektionspunkt MP wird beispielsweise mittels Polarkoordinaten bezüglich der Zentrumsachse Mv der Vorderkammer MKV definiert, unter Verwendung von strukturellen und/oder geometrisch bestimmbaren Merkmalen oder Muster MQ im Augengewebe 20 zur Bestimmung der Verdrehung um die Zentrumsachse Mv der Vorderkammer MKV, beispielsweise Merkmale und/oder Muster der Iris M24 aus einem Aufsichtsbild des Auges 2, wie obenstehend für die Bezugspunkte auf den ringförmigen Referenzstrukturen M200 beschrieben wurde.

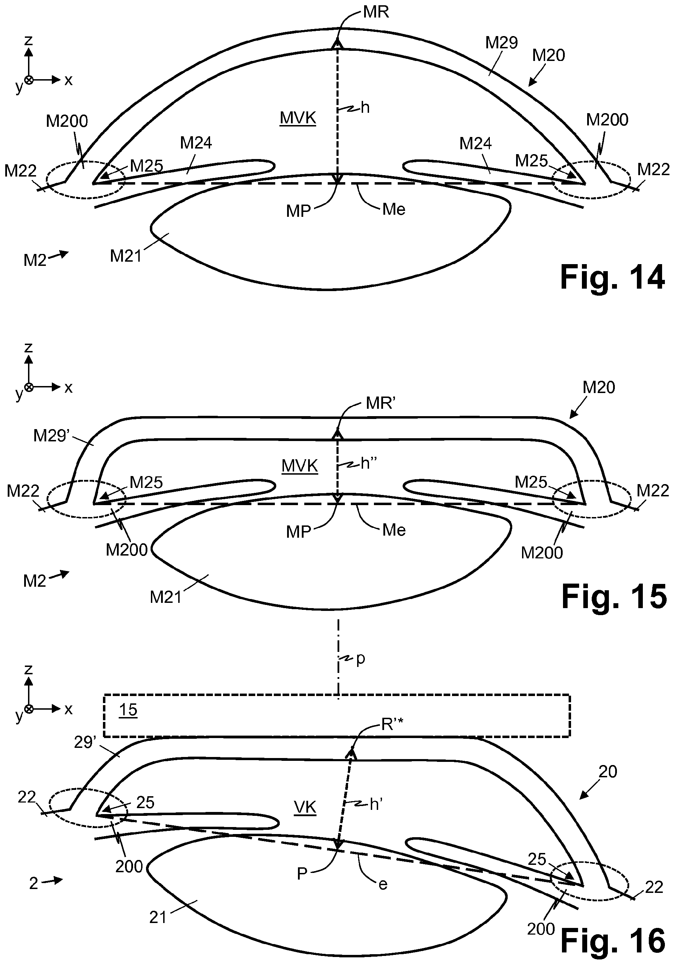

In der Figur 15 ist die zweite Ausführungsvariante angewendet auf das Augenmodell M2 im deformierten Zustand des Auges 2 schematisch im Querschnitt illustriert. Die durch die Deformation verkürzte Distanz h" wird entweder im deformierten Augenmodell M2 ermittelt oder aufgrund der durch den Kontaktkörper 15 bewirkten Deformation aus den im Augenmodell M2 des Auges 2 im nicht deformierten Zustand ermittelten Distanzangabe h berechnet. An dieser Stelle soll angeführt werden, dass die verkürzte Distanz h', h" nicht nur vom Deformationsgrad abhängt, sondern auch unterschiedliche Werte hat für unterschiedliche Varianten des Augenmodells M2 des deformierten und verkippten Auges 2 mit verschiedenen Verkippungsgraden des Auges 2.

Im Schritt S4 wird der Applikationskopf 14 auf das zu behandelnde Auge 2 aufgesetzt und mittels des Patienteninterfaces 13 am Auge 2 fixiert.

Im Schritt S5 werden im am zu behandelnden Auge 2 angedockten und fixierten Zustand des Applikationskopfes 14 respektive des Patienteninterfaces 13 (z.B. dreidimensionale) Augenstrukturen des Auges 2 optisch erfasst und digitalisiert gespeichert. Dies wird gesteuert durch den Schaltkreis 10 mittels des Messsystems 16 durchgeführt. Zum besseren Verständnis, wird nachfolgend für die erfassten (ggf. dreidimensionalen) Augenstrukturen des zu behandelnden Auges 2 im angedockten und fixierten Zustand des Applikationskopfes 14 respektive des Patienteninterfaces 13 am zu behandelnden Auge 2 der Begriff "in-situ-Augenmodell" verwendet, im Unterschied zum Augenmodell M2, welches vor der Behandlung mittels der Planungsvorrichtung 4 ermittelt und gespeichert wird. An dieser Stelle soll angemerkt werden, dass das mittels des Messsystems 16 erfasste in-situ Augenmodell und die dazugehörenden Augenstrukturen in einer Ausführungsvariante als dreidimensionales Augenmodell respektive als dreidimensionale Augenstrukturen erfasst werden, auch wenn die Figuren jeweils bloss zweidimensionale Querschnittsbilder illustrieren und in den nachfolgenden Erläuterungen nicht explizit auf ein dreidimensionales in-situ Augenmodell respektive dreidimensionale Augenstrukturen verwiesen wird.

Die Figuren 8, 10, 13, 16, 17 und 18 illustrieren schematisch im Querschnitt das Auge 2 respektive das in-situ-Augenmodell und die optisch erfassten Augenstrukturen im angedockten Zustand des Applikationskopfes 14, wobei das Auge 2 wie in der Figur 2 dargestellt bezüglich der optischen Achse p der Fokussieroptik 12 verkippt ausgerichtet ist, d.h. die Zentrumsachse v der Vorderkammer VK verläuft weder koaxial noch parallel zur optischen Achse p der Fokussieroptik 12. Dabei illustrieren die Figuren 8, 10 und 17 das Auge 2 respektive das entsprechende in-situ-Augenmodell im angedockten Zustand ohne Applikation eines Kontaktkörpers 15 und somit ohne Deformation. Die Figuren 13, 16 und 18 illustrieren das Auge 2 respektive das entsprechende in-situ-Augenmodell im angedockten Zustand mit Applikation eines Kontaktkörpers 15 und einer dadurch bewirkten Deformation des Augengewebes 20, insbesondere der Cornea 29'. Wie in der Figur 8 ersichtlich ist, umfassen die optisch erfassten Augenstrukturen des Auges 2, respektive das in-situ-Augenmodell, die Cornea 29, mit ihrer äusseren (vorderen) Cornea Oberfläche 29a und ihrer inneren (hinteren) Cornea Oberfläche 29p, die Iris 24 und die Augenlinse 21, welche die Vorderkammer VK definieren, sowie weitere Augenstrukturen im gestrichelt umrandeten Bereich des Übergangs von der Cornea 29 zur Sklera 22, an der äusseren (vorderen) Oberfläche der Cornea 29a, und des Vorderkammerwinkels 25 an der inneren (hinteren) Oberfläche der Cornea 29p, die als Referenzstrukturen 200 bestimmt und verwendet werden, wie nachfolgend erläutert wird.

Im Schritt S6 ermittelt der Schaltkreis 10 aufgrund der optisch erfassten und digitalisiert gespeicherten Augenstrukturen des Auges 2 respektive des in-situ-Augenmodells Referenzstrukturen 200 des Auges 2, welche im Wesentlichen ringförmig um die Zentrumsachse v der Vorderkammer VK angeordnet sind. Die ringförmige Anordnung um die Zentrumsachse v der Vorderkammer VK ist in der Aufsicht in den Figuren 20, 22 und 24 ersichtlich, wobei hier der Bedeutung halber angemerkt wird, dass diese Aufsicht sowohl für das Auge 2 im nicht deformierten (siehe Figur 4) als auch im angedockten, deformierten Zustand (siehe Figur 5) gilt, da die ringförmigen Referenzstrukturen 200 durch die Deformation beim Andocken im Wesentlichen unverändert bleiben (dasselbe gilt entsprechend für die Aufsicht der Figuren 19, 21, 23 und das in der Figur 6 dargestellte Augenmodell M2 im nicht deformierten und deformierten Zustand). Die ringförmigen Referenzstrukturen 200 umfassen die oben mit Bezug zur Figur 4 erwähnten und auch in den Figuren 5 und 6 detailliert wiedergegebenen Limbus 23, Iris 24, Vorderkammerwinkel 25, Skleral Spur 26, Schlemmscher Kanal 27 und/oder Schwalbes Linie 28 (in den Figuren 7-18 und 25-26 werden dieselben ringförmigen Referenzstrukturen 200 der Einfachheit halber bloss summarisch mit dem gestrichelt angedeuteten und mit dem Bezugszeichen 200 versehenen Bereich illustriert).

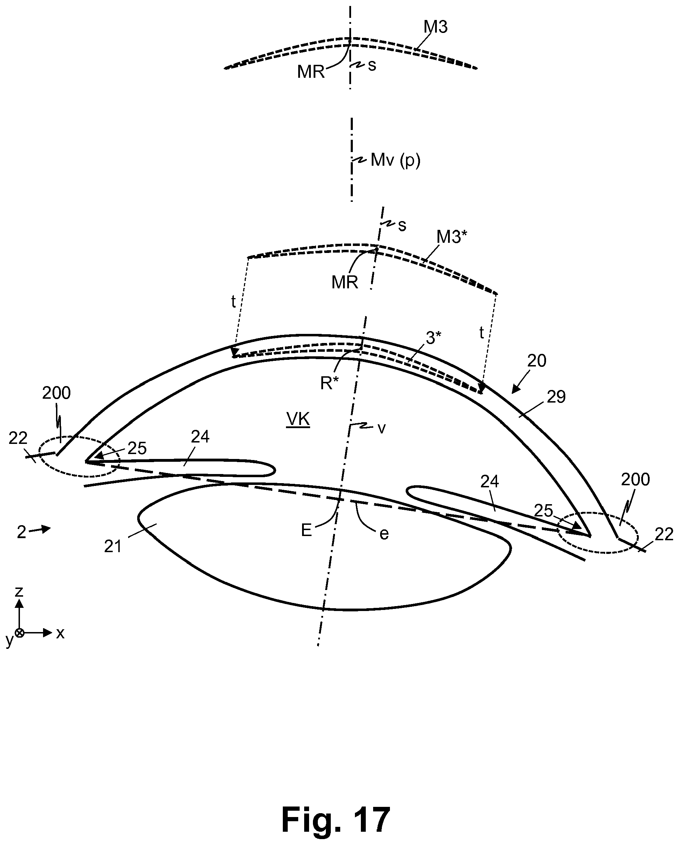

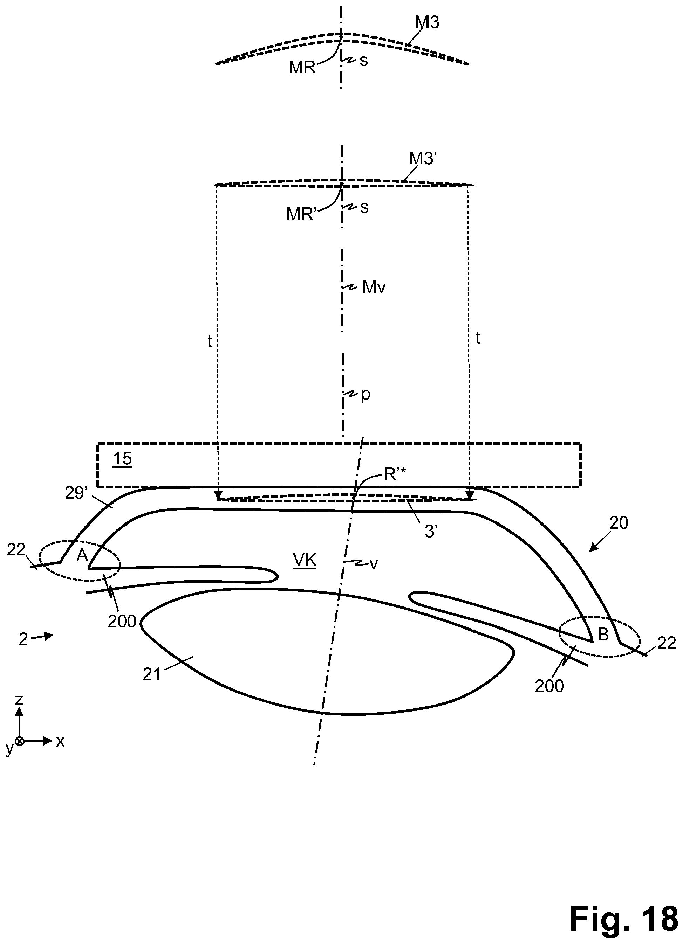

Im Schritt S7 bestimmt der Schaltkreis 10 die Ausrichtung des definierten dreidimensionalen Bearbeitungsmodells M3 zur Bearbeitung des bezüglich der optischen Achse p der Fokussieroptik 12 verkippten Auges 2, d.h. zum Bearbeiten des durch das Bearbeitungsmodell M3 respektive das deformierte Bearbeitungsmodell M3' bestimmten Bearbeitungsmusters 3, 3' im Augengewebe 20. An dieser Stelle soll festgehalten werden, dass die Bestimmung der Ausrichtung des dreidimensionalen Bearbeitungsmodells M3 nicht erforderlich ist, wenn das Bearbeitungsmodell M3 für ein Bearbeitungsmuster 3 in der Cornea 29 ausgerichtet ist und die Cornea 29 mittels des Kontaktkörpers 15 des Patienteninterfaces 13 in eine definierte Lage geformt wird, wie in den Figuren 13, 16 und 18 illustriert ist, wo das Auge 2 respektive das entsprechende in-situ-Augenmodell im angedockten Zustand dargestellt ist und das Augengewebes 20, insbesondere die Cornea 29', durch den Kontaktkörper 15 deformiert ist und eine definierte Lage zur optischen Achse p der Fokussieroptik 12 aufweist. Andernfalls, erfolgt die Ausrichtung des dreidimensionalen Bearbeitungsmodells M3 bezüglich der im angedockten Zustand des Applikationskopfs 14 ermittelten Referenzstrukturen 200 im in-situ-Augenmodell. Dabei wird das dreidimensionale Bearbeitungsmodell M3 ausgehend von einer koaxialen oder parallelen Ausrichtung seiner Zentrumsachse s zur Zentrumsachse Mv der Vorderkammer MVK im Augenmodell M2 bezüglich der im angedockten Zustand des Applikationskopfs 14 ermittelten Referenzstrukturen 200 des Auges respektive des in-situ-Augenmodells ausgerichtet. Zur Ausrichtung des dreidimensionalen Bearbeitungsmodells M3 respektive seiner Zentrumsachse s ermittelt der Schaltkreis 10 eine durch die Referenzstrukturen 200 verlaufende Referenzebene e und/oder die Zentrumsachse v der Vorderkammer VK des Auges 2 respektive des in-situ-Augenmodells, welche normal zur Referenzebene e durch einen Zentrumspunkt E der ermittelten ringförmigen Referenzstrukturen 200 des in-situ-Augenmodells verläuft (siehe Figuren 17 und 18). Zur Bearbeitung des Auges 2 im angedockten, verkippten Zustand, richtet der Schaltkreis 10 das dreidimensionale Bearbeitungsmodell M3 ausgehend von seiner initialen Ausrichtung so aus, dass die Zentrumsachse s des dreidimensionalen Bearbeitungsmodells M3 normal zur Referenzebene e respektive parallel zur Zentrumsachse v der Vorderkammer VK verläuft. Zur Illustration wird das definierte dreidimensionale Bearbeitungsmodell M3 in der Figur 17 schematisch mit seiner Zentrumsachse s dargestellt, welche parallel zur Zentrumsachse Mv der Vorderkammer MKV im Augenmodell M2 und damit zur optischen Achse p der unverkippten Fokussieroptik 12 ausgerichtet ist. Das bezüglich der Referenzebene e respektive der Zentrumsachse v der Vorderkammer VK im verkippten Auge 2 respektive im in-situ-Augenmodell ausgerichtete dreidimensionale Bearbeitungsmodell M3* ist in der Figur 17 ebenfalls schematisch dargestellt. Das Bezugszeichen 3* bezieht sich auf das im Auge 2 zu bearbeitende Bearbeitungsmuster, welches durch das verkippte, bezüglich der Referenzebene e respektive der Zentrumsachse v der Vorderkammer VK des Auges 2 ausgerichtete dreidimensionale Bearbeitungsmodell M3* definiert wird, wenn dieses an der vorgesehenen, geplanten Position R* im Auge 2 positioniert wird, wie nachfolgend erläutert wird. In der Figur 18 ist das deformierte dreidimensionale Bearbeitungsmodell M3' mit seiner Zentrumsachse s schematisch dargestellt. Das Bezugszeichen 3' bezieht sich auf das im Auge 2 zu bearbeitende deformierte Bearbeitungsmuster, welches durch das deformierte dreidimensionale Bearbeitungsmodell M3' definiert wird, wenn dieses an der vorgesehenen, geplanten Position R'* im Auge 2 respektive im in-situ-Augenmodell positioniert wird, wie nachfolgend erläutert wird.

Im Schritt S8 bestimmt der Schaltkreis 10 die Positionierung des gegebenenfalls ausgerichteten und/oder deformierten dreidimensionalen Bearbeitungsmodells M3*, M3'* zur Bearbeitung des bezüglich der optischen Achse p der Fokussieroptik 12 verkippten Auges 2. Der Schaltkreis 10 verwendet dazu den Positionierungsreferenzpunkt MR, MR' des (ggf. deformierten) dreidimensionalen Bearbeitungsmodells M3, M3' (M3*, M3'*) dessen Position über seine Referenzmessdaten m bezüglich der Referenzstrukturen M200 im Augenmodell M2 definiert ist, und bestimmt einen verschobenen (verkippten) Positionierungsreferenzpunkt R*, R'* im verkippten Auge 2 respektive im in-situ-Augenmodell. Der Schalkreis 10 ermittelt den verschobenen (verkippten) Positionierungsreferenzpunkt R*, R'* unter Verwendung der Referenzmessdaten m bezüglich der ermittelten Referenzstrukturen 200 im verkippten Auge 2 respektive im in-situ-Augenmodell.

Im optionalen Schritt S12 ermittelt der Schaltkreis 10 den Einfluss des tatsächlichen Andockvorgangs des Applikationskopf 14 mittels dessen Patienteninterface 13 an das Auge 2 auf das Bearbeitungsmodell M3 und erzeugt ein Bearbeitungsmodell M3' für den tatsächlichen angedockten Zustand. Wenn bereits ein Bearbeitungsmodell M3' für den angedockten Zustand vom funktionalen Modul der Planungsvorrichtung 4 vorliegt, erzeugt der Schaltkreis 10 ein angepasstes Bearbeitungsmodell M3' für den tatsächlichen angedockten Zustand; andernfalls erzeugt der Schalkreis 10 das Bearbeitungsmodell M3' für den tatsächlichen angedockten Zustand aufgrund des ursprünglichen Bearbeitungsmodells M3 von der Planungsvorrichtung 4. Das Auge 2 im tatsächlich angedockten Zustand des Applikationskopfes 14 respektive des Patienteninterfaces 13 am Auge 2 ist durch das im Schritt S5 in diesem Zustand ermittelte und gespeicherte in-situ-Augenmodell mit den erfassten Augenstrukturen des Auges 2 bestimmt. Das für den tatsächlichen angedockten Zustand erzeugte respektive angepasste Bearbeitungsmodell M3' ergibt sich aus dem Bearbeitungsmodell M3 respektive aus dem deformierten Bearbeitungsmodell M3' entsprechend dem Mapping des Augenmodells M2 respektive des deformierten Augenmodells M2' auf das in-situ-Augenmodell des Auges 2 im angedockten Zustand. Der Fachmann wird verstehen, dass bei der Deformation des Auges 2 durch einen Kontaktkörper 15 beim Andocken aufgrund der Steifigkeit und geringen Dehnbarkeit der Cornea 29 von einem konstanten Volumen des Auges 2 ausgegangen werden kann. Überdies bleiben die ringförmigen Referenzstrukturen 200 bei der Deformation des Auges 2 wie bereits erwähnt im Wesentlichen unverändert, so dass das Mapping des Augenmodells M2 respektive des deformierten Augenmodells M2' auf das in-situ-Augenmodell des Auges 2 im angedockten Zustand aufgrund der Abmessungen der ringförmigen Referenzstrukturen 200 (im ursprünglich erfassten Augenmodell M2 und im in-situ-Augenmodell des Auges 2 im angedockten Zustand) skalierbar ist. In einer Ausführungsvariante skaliert der Schaltkreis 10 das Augenmodell M2 respektive das deformierte Augenmodell M2' und das darin positionierte Bearbeitungsmodel M3 des zu bearbeitenden Bearbeitungsmusters 3 aufgrund der ringförmigen Referenzstrukturen M200, 200 auf die Grösse des Auges 2 im angedockten (deformierten) Zustand gemäss dem in-situ-Augenmodell. Danach bestimmt der Schaltkreis 10 eine Mapping-Transformation zur Überführung des Augenmodells M2 respektive des deformierten Augenmodells M2' auf das in-situ-Augenmodell des Auges 2 im angedockten (deformierten) Zustand, beispielsweise auf der Basis einer Aufteilung des Augenmodells M2 respektive des deformierten Augenmodells M2' und des in-situ-Augenmodells des Auges 2 im angedockten (deformierten) Zustand in eine endliche Anzahl entsprechender Elemente (z.B. mit Hilfe der Finite-Elemente-Methode), wobei die Mapping-Transformation jedes Element im Augenmodell M2 respektive im deformierten Augenmodell M2' einem entsprechenden (transformierten) Element im in-situ-Augenmodell des Auges 2 im angedockten (deformierten) Zustand transformiert. Der Schaltkreis 10 ermittelt das Bearbeitungsmodell M3' im angedockten (deformierten) Zustand des Auges 2 aus dem Bearbeitungsmodel im skalierten Augenmodel M2 respektive im skalierten deformierten Augenmodell M2' aufgrund der entsprechenden transformierten Elemente im in-situ-Augenmodell des Auges 2 im angedockten (deformierten) Zustand.

Der Fachmann wird verstehen, dass die Schritte S7, zum Ausrichten des Bearbeitungsmodells M3, und S8, zum Positionieren des ausgerichteten Bearbeitungsmodells M3*, auch in umgekehrter Reihenfolge ausgeführt werden können, so dass zunächst das Bearbeitungsmodell M3 positioniert wird und danach das positionierte Bearbeitungsmodell bezüglich der Referenzstrukturen 200 ausgerichtet wird.

Gemäss der ersten Ausführungsvariante, in welcher die Referenzmessdaten m Distanzgaben zur Bestimmung der Position des Positionierungsreferenzpunkts MR, MR' im Augenmodell M2 umfassen, werden basierend auf den im Augenmodell M2 erfassten Bezugspunkten MA, MB, die entsprechenden Bezugspunkte A, B auf den ermittelten ringförmigen Referenzstrukturen 200 des verkippten Auges 2 im in-situ-Augenmodell bestimmt, beispielsweise unter Verwendung der strukturellen und/oder geometrisch bestimmbaren Merkmale oder Muster Q im Augengewebe 20 zur Bestimmung der Verdrehung um die Zentrumsachse v der Vorderkammer VK, wie obenstehend im Bezug zur Figur 19 beschrieben wurde und schematisch in der Figur 20 illustriert ist. In den Ausführungsvarianten gemäss den Figuren 21, 23 mit mehreren im Augenmodell M2 (z.B. in Drittel- respektive Viertel-Sektoren) verteilten Bezugspunkten MD, ME, MF, MG, MH, MJ, MK werden die entsprechenden Bezugspunkte D, E, F, G, H, J, K auf den ermittelten ringförmigen Referenzstrukturen 200 des verkippten Auges 2 im in-situ-Augenmodell bestimmt, wie in den Figuren 20, 22, 24 schematisch illustriert ist.

Wie in der Figur 8 im Querschnitt und in der Figur 20 in der Aufsicht schematisch illustriert ist, wird der verschobene (verkippte) Positionierungsreferenzpunkt R* ausgehend von den bestimmten Bezugspunkten A, B, D, E, F, G, H, J, K auf den ermittelten ringförmigen Referenzstrukturen 200 des verkippten Auges 2 im in-situ-Augenmodell unter Verwendung der für das Augenmodell M2 gespeicherten Distanzen a, b, d, e, f, g, h, j, k, q, r zum Positionierungsreferenzpunkt MR ermittelt, jeweils ausgehend von den Bezugspunkten A, B, D, E, F, G, H, J, K im in-situ Augenmodell als Wegstrecken a, b, d, e, f, g, h, j, k entlang der entsprechenden Augenstrukturen im in-situ Augenmodell, respektive bei direkten Strecken q, r als Schnittpunkt von Kugelschalen um die Bezugspunkte A, B, D, E, F, G, H, J, K mit dem Radius der Strecken m, n. Im Beispiel der Figur 8, wird der verschobene (verkippte) Positionierungsreferenzpunkt R* ausgehend von den bestimmten Bezugspunkten A, B im in-situ-Augenmodell unter Verwendung der für das Augenmodell M2 gespeicherten Wegstrecken a, b entlang der neutralen Faser 29n in der Cornea 29 des in-situ-Augenmodells bestimmt.

Die Figur 13 illustriert die Bestimmung des verschobenen (verkippten) Positionierungsreferenzpunkt R'* beim deformierten Auge 2 am Beispiel der für das Augenmodell M2 erfassten und gespeicherten 'Wegstrecken a, b, ausgehend von den bestimmten Bezugspunkten A, B auf den ringförmigen Referenzstrukturen 200 des verkippten Auges 2 im in-situ-Augenmodell. Die Wegstrecken a, b werden im in-situ-Augenmodell des deformierten Auges 2 entlang der neutralen Faser 29n' in der deformierten Cornea 29' geführt. Abhängig von der Genauigkeit bei der Bestimmung der neutralen Faser 29n' in der deformierten Cornea 29' oder wenn die Wegstrecken a, b im Augenmodell M2 entlang der äusseren und/oder inneren Corneaoberflächen 29a, 29p bestimmt und im in-situ Augenmodell entsprechend auf die äussere und/oder innere Corneaoberflächen 29p', 29a' der deformierten Cornea 29' übertragen werden, wird der verschobene (verkippte) Positionierungsreferenzpunkt R'* durch das gleichbleibende Verhältnis a:b der Wegstrecken bestimmt. Auch hier gilt wiederum, das nicht bloss die 'Wegstrecken a, b zu zwei gegenüberliegenden Bezugspunkten auf den ringförmigen Referenzstrukturen 200 verwendet werden können, sondern dazu Wegstrecken entlang von Augenstrukturen zu mehreren Bezugspunkten MA, MB, MC, MD, ME, MF, MG, MH, MJ, MK und/oder direkte Distanzen q, r verwendet werden können.

Gemäss der zweiten Ausführungsvariante, in welcher die Referenzmessdaten m einen Projektionspunkt MP des Positionierungsreferenzpunkts MR auf der durch die ringförmigen Referenzstrukturen M200 definierten Referenzebene Me und eine Distanzangabe h des Positionierungsreferenzpunkts MR zur Referenzebene Me respektive zum Projektionspunkt MP umfassen, wird basierend auf dem im Augenmodell M2 erfassten Projektionspunkt MP der entsprechende Projektionspunkt P auf der durch die ermittelten ringförmigen Referenzstrukturen 200 des verkippten Auges 2 im in-situ Augenmodell definierte Referenzebene e bestimmt, beispielsweise unter Verwendung der strukturellen und/oder geometrisch bestimmbaren Merkmale oder Muster Q im Augengewebe 20 zur Bestimmung der Verdrehung um die Zentrumsachse v der Vorderkammer VK, wie obenstehend im Zusammenhang mit der Bestimmung der Bezugspunkte A, B, D, E, F, G, H, J, K angeführt wurde.

Wie in der Figur 10 im Querschnitt schematisch illustriert ist, wird der verschobene (verkippte) Positionierungsreferenzpunkt R* ausgehend vom bestimmten Projektionspunkt P auf der durch die ringförmigen Referenzstrukturen 200 des verkippten Auges 2 im in-situ Augenmodell definierten Referenzebene e unter Verwendung der für das Augenmodell M2 gespeicherte Distanz h zum Positionierungsreferenzpunkt MR ermittelt, als Abstandspunkt mit der Distanz h auf einer Normalen zur Referenzebene e durch den Projektionspunkt P. Die Figur 16 illustriert dasselbe entsprechend für das deformierte Auge 2, wobei der verschobene (verkippte) Positionierungsreferenzpunkt R'* im deformierten Augengewebe 20, insbesondere in der deformierten Cornea 29', im in-situ Augenmodell mittels der durch die Deformation verkürzten Distanz h' bestimmt wird.

Wie in der Figur 17 illustriert ist, wird das im Schritt S7 ausgerichtete Bearbeitungsmodell M3* zum Bearbeiten des Bearbeitungsmusters 3* durch den Schaltkreis 10 am verschobenen (verkippten) Positionierungsreferenzpunkt R* positioniert, das heisst der Positionierungsreferenzpunkt MR des ausgerichteten Bearbeitungsmodells M3* wird am bestimmten Positionierungsreferenzpunkt R* positioniert, wie durch die gestrichelten Pfeile t schematisch illustriert ist.

Wie in der Figur 18 illustriert ist, wird das im Schritt S11 und/oder S12 deformierte Bearbeitungsmodell M3' zum Bearbeiten des Bearbeitungsmusters 3' durch den Schaltkreis 10 am verschobenen (verkippten) Positionierungsreferenzpunkt R'* positioniert, das heisst der Positionierungsreferenzpunkt MR' des deformierten Bearbeitungsmodells M3' wird am bestimmten Positionierungsreferenzpunkt R'* positioniert, wie durch die gestrichelten Pfeile t schematisch illustriert ist.

Im Schritt S9 steuert der Schaltkreis 10 die Laserquelle 11 und das Scannersystem 17 im am Auge 2 fixierten Zustand des Applikationskopfs 14 so, dass das Scannersystem 17 den von der Laserquelle 11 erzeugten gepulsten Laserstrahl L gemäss dem bezüglich der Referenzstrukturen 200 positionierten und gegebenenfalls ausgerichteten (deformierten respektive nicht deformierten) dreidimensionalen Bearbeitungsmodell M3*, M3' auf die Bearbeitungszielpunkte F des im Auge 2 zu bearbeitenden dreidimensionalen Bearbeitungsmusters 3*, 3' lenkt.

## Patentansprüche

1. Ophthalmologische Vorrichtung (1) zum Bearbeiten eines Auges (2), umfassend eine Laserquelle (11), die eingerichtet ist, einen gepulsten Laserstrahl (L) zu erzeugen; einen Applikationskopf (14) mit einer Fokussieroptik (12) und einem Patienteninterface (13), wobei die Fokussieroptik (12) eingerichtet ist, den gepulsten Laserstrahl (L) im Auge (2) zu fokussieren, und wobei das Patienteninterface (13) eingerichtet ist, den Applikationskopf (14) am Auge (2) zu fixieren; einen Schaltkreis (10), der eingerichtet ist, ein dreidimensionales Bearbeitungsmodell (M3) eines im Auge (2) zu bearbeitenden dreidimensionalen Bearbeitungsmusters (3) zu speichern; ein Scannersystem (17), das eingerichtet ist, den gepulsten Laserstrahl (L) gemäss dem dreidimensionalen Bearbeitungsmodell (M3) auf Bearbeitungszielpunkte (F) des im Auge (2) zu bearbeitenden dreidimensionalen Bearbeitungsmusters (3) zu lenken; und ein Messsystem (16), das eingerichtet ist, in einem am Auge (2) fixierten Zustand des Applikationskopfs (14) Strukturen des Auges (2) optisch zu erfassen; **dadurch gekennzeichnet,**
**dass** der Schaltkreis (10) eingerichtet ist, aufgrund der durch das Messsystem (16) im am Auge (2) fixierten Zustand des Applikationskopfs (14) optisch erfassten Strukturen Referenzstrukturen (200) des Auges (2) zu ermitteln, welche im Wesentlichen ringförmig um eine Zentrumsachse (v) der Vorderkammer (VK) des Auges (2) angeordnet sind, und
**dass** der Schaltkreis (10) eingerichtet ist, das dreidimensionale Bearbeitungsmodell (M3) bezüglich der Referenzstrukturen (200) auszurichten, und das Scannersystem (17) im am Auge (2) fixierten Zustand des Applikationskopfs (14) derart zu steuern, dass das Scannersystem (17) den gepulsten Laserstrahl (L) gemäss dem bezüglich der Referenzstrukturen (200) ausgerichteten dreidimensionalen Bearbeitungsmodell auf die Bearbeitungszielpunkte (F) des im Auge (2) zu bearbeitenden dreidimensionalen Bearbeitungsmusters (3*) lenkt.

2. Ophthalmologische Vorrichtung (1) nach Anspruch 1, **dadurch gekennzeichnet, dass** der Schaltkreis (10) eingerichtet ist, einen Positionierungsreferenzpunkt (MR), zur Positionierung des dreidimensionalen Bearbeitungsmodells (M3) im Auge (2), zu speichern, unter Verwendung der Referenzstrukturen (200) einen im am Auge (2) fixierten Zustand des Applikationskopfs (14) verschobenen Positionierungsreferenzpunkt (R*) zu bestimmen, das dreidimensionale Bearbeitungsmodell (M3) im am Auge (2) fixierten Zustand des Applikationskopfs (14) bezüglich des verschobenen Positionierungsreferenzpunkts (R*) zu positionieren, und das Scannersystem (17) im am Auge (2) fixierten Zustand des Applikationskopfs (14) derart zu steuern, dass das Scannersystem (17) den gepulsten Laserstrahl (L) gemäss dem bezüglich des verschobenen Positionierungsreferenzpunkts (R*) positionierten dreidimensionalen Bearbeitungsmodell (M3) auf die Bearbeitungszielpunkte (F) des im Auge (2) zu bearbeitenden dreidimensionalen Bearbeitungsmusters (3*) lenkt.

3. Ophthalmologische Vorrichtung (1) nach Anspruch 2, **dadurch gekennzeichnet, dass** der Schaltkreis (10) eingerichtet ist, den Positionierungsreferenzpunkt (MR) mit Referenzmessdaten (m) zur Positionierung des Positionierungsreferenzpunkts bezüglich der Referenzstrukturen (M200) in einem nicht am Auge (2) fixierten Zustand des Applikationskopfs (14) zu speichern, und den verschobenen Positionierungsreferenzpunkt (R*) im am Auge (2) fixierten Zustand des Applikationskopfs (14) unter Verwendung der Referenzmessdaten (m) und der im am Auge (2) fixierten Zustand des Applikationskopfs (14) ermittelten Referenzstrukturen (200) zu bestimmen.

4. Ophthalmologische Vorrichtung (1) nach Anspruch 3, **dadurch gekennzeichnet, dass** die Referenzmessdaten (m) Distanzangaben (a, b) vom Positionierungsreferenzpunkt (MR) zu mindestens zwei gegenüberliegenden Punkten (A, B) auf den im Wesentlichen ringförmig angeordneten Referenzstrukturen (M200) im nicht am Auge (2) fixierten Zustand des Applikationskopfs (14) umfassen, und dass der Schaltkreis (10) eingerichtet ist, den verschobenen Positionierungsreferenzpunkt (R*) im am Auge (2) fixierten Zustand des Applikationskopfs (14) unter Verwendung der Distanzangaben (a, b) bezüglich der mindestens zwei gegenüberliegenden Punkten (A, B) auf den im am Auge (2) fixierten Zustand des Applikationskopfs (14) ermittelten Referenzstrukturen (200) zu bestimmen.

5. Ophthalmologische Vorrichtung (1) nach Anspruch 4, **dadurch gekennzeichnet, dass** die Distanzangaben (a, b) Weglängen vom Positionierungsreferenzpunkt (R) entlang erfasster Augenstrukturen des Auges (2) zu den mindestens zwei gegenüberliegenden Punkten (A, B) auf den im Wesentlichen ringförmig angeordneten Referenzstrukturen (200) definieren.

6. Ophthalmologische Vorrichtung (1) nach Anspruch 5, **dadurch gekennzeichnet, dass** die Distanzangaben (a, b) Weglängen entlang mindestens einem umfassen aus: neutrale Faser (29n) der Cornea (29), Corneavorderfläche (29a) und Cornearückfläche (29p).

7. Ophthalmologische Vorrichtung (1) nach einem der Ansprüche 3 bis 6, **dadurch gekennzeichnet, dass** der Schaltkreis (10) eingerichtet ist, den verschobenen Positionierungsreferenzpunkt (R*) im am Auge (2) fixierten Zustand des Applikationskopfs (14) unter Verwendung eines Verhältnisses der in den Referenzmessdaten (m) enthaltenen Distanzangaben (a, b) zu bestimmen.

8. Ophthalmologische Vorrichtung (1) nach Anspruch 3, **dadurch gekennzeichnet, dass** die Referenzmessdaten (m) eine Distanzangabe (h) und einen Projektionspunkt (MP) des Positionierungsreferenzpunkts (MR) bezüglich einer Referenzebene (Me) umfassen, welche durch im nicht am Auge (2) fixierten Zustand des Applikationskopfs (14) erfasste Referenzstrukturen (M200) definiert ist; und dass der Schaltkreis (10) eingerichtet ist, den verschobenen Positionierungsreferenzpunkt (R*) im am Auge (2) fixierten Zustand des Applikationskopfs (14) unter Verwendung der Distanzangabe (h) und des Projektionspunkts (P) des Positionierungsreferenzpunkts (R) bezüglich der Referenzebene (e) im am Auge (2) fixierten Zustand des Applikationskopfs (14) zu bestimmen, definiert durch die im am Auge (2) fixierten Zustand des Applikationskopfs (14) erfassten Referenzstrukturen (200).

9. Ophthalmologische Vorrichtung (1) nach einem der Ansprüche 1 bis 8, **dadurch gekennzeichnet, dass** der Schaltkreis (10) eingerichtet ist, aufgrund der ermittelten Referenzstrukturen (200) eine durch die Referenzstrukturen (200) definierte Zentrumsachse (v) der Vorderkammer (VK) zu bestimmen, und das dreidimensionale Bearbeitungsmodell (M3) bezüglich der Zentrumsachse (v) der Vorderkammer (VK) auszurichten.

10. Ophthalmologische Vorrichtung (1) nach einem der Ansprüche 1 bis 9, **dadurch gekennzeichnet, dass** der Schaltkreis (10) eingerichtet ist, eine durch die Referenzstrukturen (200) verlaufende Referenzebene (e) zu bestimmen, und das dreidimensionale Bearbeitungsmodell (M3) bezüglich der Referenzebene (e) auszurichten.

11. Ophthalmologische Vorrichtung (1) nach einem der Ansprüche 1 bis 10, **dadurch gekennzeichnet, dass** die Referenzstrukturen (200) mindestens eines umfassen aus: Limbus (23), Iris (24), Vorderkammerwinkel (25), Skleral Spur (26), Schlemmscher Kanal (27) und Schwalbes Linie (28).

12. Ophthalmologische Vorrichtung (1) nach einem der Ansprüche 1 bis 11, **dadurch gekennzeichnet, dass** der Schaltkreis (10) eingerichtet ist, das dreidimensionale Bearbeitungsmodell (M3) über eine Kommunikationsstrecke (5) von einer externen Planungsvorrichtung (4) zu empfangen.

13. Ophthalmologische Vorrichtung (1) nach einem der Ansprüche 1 bis 12, **dadurch gekennzeichnet, dass** das Messsystem (16) eingerichtet ist, die Strukturen des Auges (2) über einen in Richtung einer optischen Achse (p) der Fokussieroptik (12) verlaufenden Tiefenbereich optisch zu erfassen.

14. Ophthalmologische Vorrichtung (1) nach einem der Ansprüche 1 bis 13, **dadurch gekennzeichnet, dass** das Messsystem (16) als interferometrisches Messsystem ausgeführt ist, und dass der Schaltkreis (10) eingerichtet ist, das Messsystem (16) derart zu steuern, dass das Messsystem (16) im am Auge (2) fixierten Zustand des Applikationskopfs (14) die Strukturen (200) des Auges (2) optisch erfasst, und die Referenzstrukturen (200) aus den optisch erfassten Strukturen (200) zu ermitteln.

15. Ophthalmologische Vorrichtung (1) nach einem der Ansprüche 1 bis 14, **dadurch gekennzeichnet, dass** das Messsystem (16) eingerichtet ist, die Strukturen des Auges (2) in einem oder mehreren Querschnittsbildern des Auges (2) zu erfassen, und dass der Schaltkreis (10) eingerichtet ist, die Referenzstrukturen (200) des Auges (2) aus dem einen oder den mehreren Querschnittsbildern des Auges (2) zu ermitteln.

16. Ophthalmologische Vorrichtung (1) nach einem der Ansprüche 1 bis 15, **dadurch gekennzeichnet, dass** das Patienteninterface (13) einen Kontaktkörper (15) umfasst, welcher im am Auge (2) fixierten Zustand des Applikationskopfs (14) auf dem Auge (2) aufliegt und das Auge (2) deformiert; und dass der Schaltkreis (10) eingerichtet ist, das dreidimensionale Bearbeitungsmodell (M3) in ein deformiertes dreidimensionales Bearbeitungsmodell (M3') eines im Auge (2) zu bearbeitenden deformierten dreidimensionalen Bearbeitungsmusters (3') zu transformieren, unter Verwendung der Referenzstrukturen (200) einen im am Auge (2) fixierten Zustand des Applikationskopfs (14) verschobenen Positionierungsreferenzpunkt (R'*) zu bestimmen, und das deformierte dreidimensionale Bearbeitungsmodell (M3')im am deformierten Auge (2) fixierten Zustand des Applikationskopfs (14) bezüglich des verschobenen Positionierungsreferenzpunkt (R'*) zu positionieren, und das Scannersystem (17) im am deformierten Auge (2) fixierten Zustand des Applikationskopfs (14) derart zu steuern, dass das Scannersystem (17) den gepulsten Laserstrahl (L) gemäss dem bezüglich des verschobenen Positionierungsreferenzpunkt (R'*) positionierten deformierten dreidimensionalen Bearbeitungsmodell (M3'*) auf die Bearbeitungszielpunkte (F) des im Auge (2) zu bearbeitenden deformierten dreidimensionalen Bearbeitungsmusters (3') lenkt.
